(19) Europäisches Patentamt
European Patent Office
Office européen des brevets

(11) Veröffentlichungsnummer: **0 221 502**
**A1**

(12) **EUROPÄISCHE PATENTANMELDUNG**

(21) Anmeldenummer: **86114988.8**

(22) Anmeldetag: **28.10.86**

(51) Int. Cl.[4]: **C07C 121/43**, C07C 103/46, C07C 103/737, C07C 101/36, C07C 120/00, C07C 102/00, C07C 99/00, C07D 307/30, A01N 37/00

(30) Priorität: **06.11.85 DE 3539307**

(43) Veröffentlichungstag der Anmeldung: **13.05.87 Patentblatt 87/20**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Lunkenheimer, Winfried, Dr.**
**Bismarckstrasse 29**
**D-5600 Wuppertal 1(DE)**
Erfinder: **Brandes, Wilhelm, Dr.**
**Eichendorffstrasse 3**
**D-5653 Leichlingen 1(DE)**

(54) **Substituierte 1-Amino-cyclopropancarbonsäurederivate.**

(57) Substituierte 1-Amino-cyclopropancarbonsäurederivate der Formel (I)

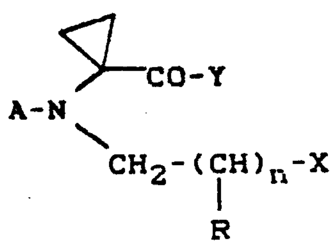

(I)

in welcher
Y, A, R, X und n die in der Beschreibung gegebenen Bedeutungen haben und ihre Verwendung zur Schädlingsbekämpfung.

Die Verbindungen können nach verschiedenen Verfahren hergestellt werden, so z. B. durch Umsetzung von geeigneten 1-Amino-cyclopropancarbonsäurederivaten mit Alkylierungsmitteln bzw. Olefinen.

EP 0 221 502 A1

## Substituierte 1-Amino-cyclopropancarbonsäurederivate

Die Erfindung betrifft neue substituierte 1-Amino-cyclopropancarbonsäurederivate, mehrere Verfahren zu ihrer Herstellung und ihre Verwendung als Schädlingsbekämpfungsmittel, insbesondere als Fungizide.

Es ist bereits bekannt, daß zahlreiche 1-Methylaminocyclopropancarbonsäure-Derivate, wie beispielsweise 1-Methylamino-cyclopropancarbonsäure, 1-(N-Fomyl-N-methylamino)-cyclopropancarbonsäure und 1-(N-Formyl-N-methylamino)-cyclopropancarbonsäureemethylester im Pflanzenschutz eingesetzt wrden können (vgl. DE-OS 3 335 514).

Es wurden neue substituierte 1-Amino-cyclopropancarbonsäurederivate der allgemeinen Formel (I)

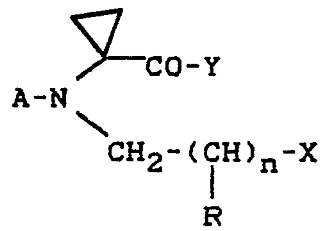

(I)

in welcher
Y für die Gruppierung $OR^1$ oder $-NR^2R^3$ steht, wobei
$R^1$ für Wasserstoff, Alkyl oder für gegebenenfalls substituiertes Benzyl steht,
$R^2$ für Wasserstoff oder Alkyl steht,
$R^3$ für Wasserstoff oder Alkyl steht, oder
$R^2$ und $R^3$ gemeinsam mit dem Stickstoffatom, an das sie gebunden sind, für einen gegebenenfalls substituierten mehrgliedrigen Ring stehen, der weitere gleiche oder verschiedene Heteroatome, wie Sauerstoff oder Stickstoff, enthalten kann,
A für Wasserstoff oder einen Acylrest steht,
R für Wasserstoff oder Alkyl steht,
n für die Zahlen 0,1 oder 2 steht, und
X für Alkenyl, Alkinyl, für Hydroxycarbonyl, Alkoxycarbonyl, Aminocarbonyl, Alkylaminocarbonyl, Dialkylaminocarbonyl oder Cyano steht,
und deren pflanzenverträglichen Säureadditionssalze und deren physiologisch verträglichen gegebenenfalls substituierten Ammonium-, Alkali-und Erdalkali-Salze gefunden.

Weiterhin wurde gefunden, daß sich die neuen substituierten 1-Amino-cyclopropancarbonsäurederivate der allgemeinen Formel (I),

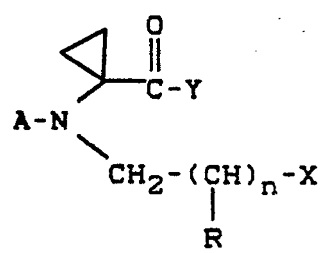

(I)

in welcher
Y für die Gruppierung $OR^1$ oder $-NR^2R^3$ steht, wobei
$R^1$ für Wasserstoff, Alkyl oder für gegebenenfalls substituiertes Benzyl steht,
$R^2$ für Wasserstoff oder Alkyl steht und
$R^3$ für Wasserstoff oder Alkyl steht, oder
$R^2$ und $R^3$ gemeinsam mit dem Stickstoffatom, an das sie gebunden sind, für einen gegebenenfalls substituierten mehrgliedrigen Ring stehen, der weitere gleiche oder verschiedene Heteroatome, wie Sauerstoff oder Stickstoff, enthalten kann,
A für Wasserstoff oder einen Acylrest steht,

R für Wasserstoff oder Alkyl steht,

n für die Zahlen 0,1 oder 2 steht und

X für Alkenyl, Alkinyl, für Hydroxycarbonyl, Alkoxycarbonyl, Aminocarbonyl, Alkylaminocarbonyl, Dialkylaminocarbonyl oder Cyano steht,

und deren pflanzenverträglichen Säureadditionssalze und gegebenenfalls substituierten Ammonium-, Alkali- und Erdalkali-Salze, nach folgenden Verfahren herstellen lassen:

Man erhält

a) die erfindungsgemäßen substituierten 1-Amino-cyclopropancarbonsäurederivate der Formel (I-1)

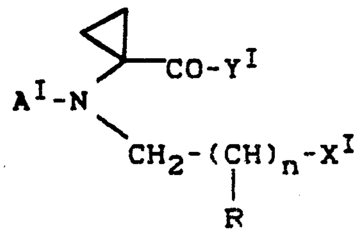

(I-1)

in welcher

R und n die oben angegebene Bedeutungen haben,

$A^I$ für einen Acylrest steht,

$Y^I$ für die Gruppierung $OR^4$ oder $-NR^5R^6$ steht, wobei

$R^4$ für Alkyl oder gegebenenfalls substituiertes Benzyl steht,

$R^5$ für Alkyl steht, und

$R^6$ für Alkyl steht, oder

$R^5$ und $R^6$ gemeinsam mit dem Stickstoffatom, an das sie gebunden sind, für einen gegebenenfalls substituierten mehrgliedrigen Ring stehen, der weitere gleiche oder verschiedene Heteroatome, wie Sauerstoff oder Stickstoff, enthalten kann,

$X^I$ für Alkenyl, Alkinyl, Alkoxycarbonyl, Dialkylaminocarbonyl oder Cyano steht,

wenn man 1-Amino-cyclopropancarbonsäurederivate der Formel (II)

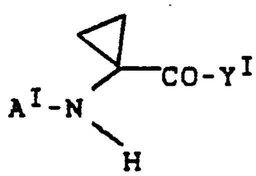

(II)

in welcher $A^I$ und $Y^I$ die oben angegebenen Bedeutungen haben, mit Verbindungen der Formel (III)

$Z-CH_2-(\underset{R}{C}H)_n-X^I$ (III)

in welcher R, n und $X^I$ die oben angegebenen Bedeutungen haben, und

Z für eine Abgangsgruppe, wie beispielsweise Chlor, Brom, Iod, Mesyloxy oder Tosyloxy, steht,

in Gegenwart einer Base, gegebenenfalls in Gegenwart eines Lösungsmittels und gegebenenfalls in Gegenwart eines Phasentransferkatalysators umsetzt,

oder man erhält

b) die erfindungsgemäßen substituierten 1-Amino-cyclopropancarbonsäurederivate der Formel (I-2)

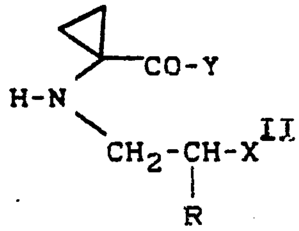

(I-2)

in welcher
R und Y die oben angegebene Bedeutungen haben, und
$X^{II}$ für Alkoxycarbonyl oder Cyano steht,
wenn man 1-Amino-cyclopropancarbonsäurederivate der Formel (IV)

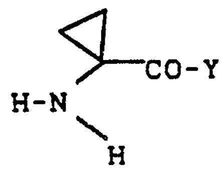

(IV)

in welcher Y die oben angegebene Bedeutung hat, mit Olefinen der Formel (V)
$H_2C = \underset{R}{C}H-X^{II}$ (V)
in welcher $X^{II}$ und R die oben angegebenen Bedeutungen haben,
gegebenenfalls in Gegenwart eines Lösungsmittels und gegebenenfalls in Gegenwart eines Katalysators umsetzt,
oder man erhält

c) die erfindungsgemäßen substituierten 1-Amino-cyclopropancarbonsäurederivate der Formel (I-3)

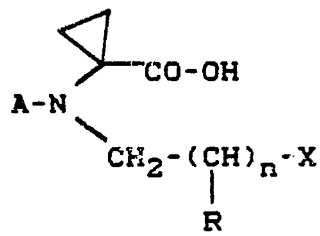

(I-3)

in welcher
A, R, n und X die oben angegebenen Bedeutungen haben,
wenn man erfindungsgemäße 1-Amino-cyclopropancarbonsäurebenzylester der Formel (I-4)

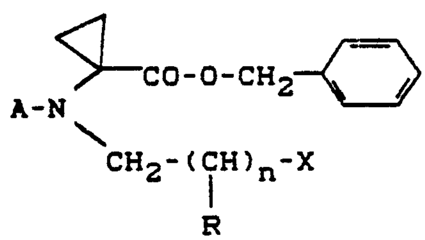

(I-4)

in welcher A, R, n und X die oben angegebenen Bedeutungen haben,
mit Wasserstoff in Gegenwart eines Katalysators und in Gegenwart eines Lösungsmittels hydriert,
oder man erhält

d) die erfindungsgemäßen substituierten 1-Amino-cyclopropancarbonsäurederivate der Formel (I-5)

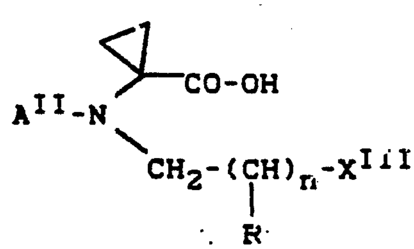

(I-5)

in welcher R und n die oben angegebenen Bedeutungen haben,
$A^{II}$ für die Bedeutung von A mit Ausnahme von Trifluoracetyl steht und
$X^{III}$ für die Bedeutung von X steht mit Ausnahme von primärem und sekundärem Alkoxycarbonyl,
wenn man erfindungsgemäße substituierte 1-Amino-cyclopropancarbonsäureester der Formel (I-6)

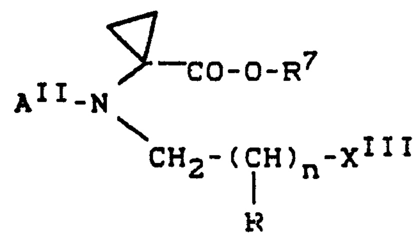

(I-6)

in welcher $A^{II}$, R, n und $X^{III}$ die oben angegebenen Bedeutungen haben und
$R^7$ für Methyl oder Ethyl steht,
in Gegenwart einer wäßrigen Base und gegebenenfalls eines Verdünnungsmittels hydrolysiert,
oder man erhält

e) die erfindungsgemäßen substituierten 1-Amino-cyclopropancarbonsäurederivate der Formel (I-7)

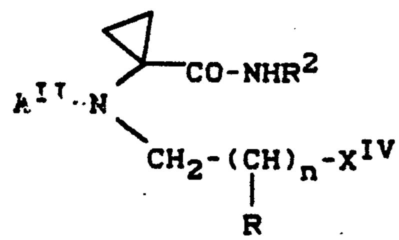

(I-7)

in welcher $A^{II}$, $R^2$, R und n die oben angegebenen Bedeutungen haben und
$X^{IV}$ für die Bedeutung von mit Ausnahme von primären Alkoxycarbonyl steht,
wenn man erfindungsgemäße substituierte 1-Amino-cyclopropancarbonsäureester der Formel (I-8)

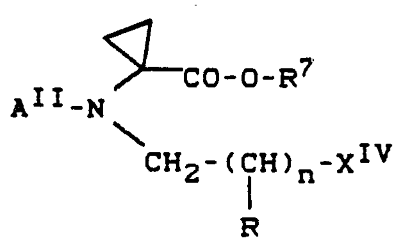

(I-8)

in welcher $A^{II}$, $R^7$, R, n und $X^{IV}$ die oben angegebenen Bedeutungen haben,
mit Aminen der Formel (VI)
$H_2NR^2$ (VI)
in welcher $R^2$ die oben angegebene Bedeutung hat,
in Gegenwart eines Lösungsmittels umsetzt,
oder man erhält

f) die erfindungsgemäßen substituierten 1-Amino-cyclopropancarbonsäurederivate der Formel (I-9)

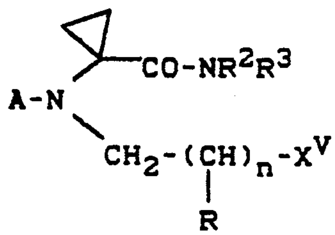

(I-9)

in welcher A, R, $R^2$, $R^3$ und n die oben angegebenen Bedeutungen haben und $X^V$ für die Bedeutung von X mit Ausnahme von Hydroxycarbonyl steht, wenn man carboxy-aktivierte Derivate der erfindungsgemäßen 1-Amino-cyclopropancarbonsäuren der Formel (I-10)

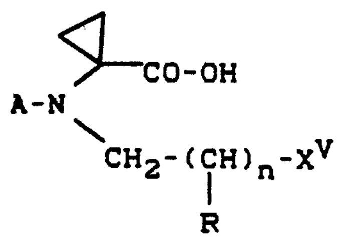

(I-10)

in welcher A, R, n und $X^V$ die oben angegebenen Bedeutungen haben, mit Aminen der Formel (VII)

$HNR^2R^3$ (VII)

in welcher $R^2$ und $R^3$ die oben angegebenen Bedeutungen haben, gegebenenfalls in Gegenwart einer Base, in Gegenwart eines Lösungmittels und gegebenenfalls in Gegenwart eines Katalysators umsetzt, oder man erhält

g) die erfindungsgemäßen substituierten 1-Amino-cyclopropancarbonsäurederivate der Formel (I-11)

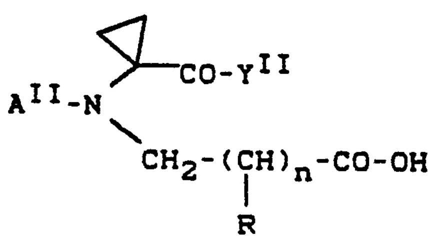

(I-11)

in welcher

$A^{II}$, R und n die oben angegebenen Bedeutungen haben und $Y^{II}$ für tert.-Alkoxy oder die Gruppierung $NR^2R^3$ steht, wobei $R^2$ und $R^3$ die oben angegebenen Bedeutungen haben, wenn man die Ester der Formel (I-12)

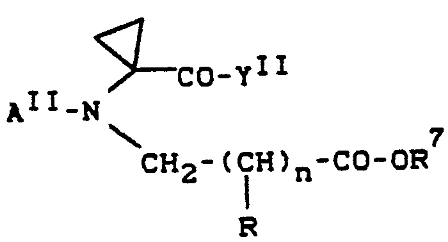

(I-12)

in welcher A ", Y", R, n und R' die oben angegebenen Bedeutungen haben, in Gegenwart einer wäßrigen Base und gegebenenfalls in Gegenwart eines Verdünnungsmittels hydrolysiert, oder man erhält

h) die erfindungsgemäßen substituierten 1-Amino-cyclopropancarbonsäurederivate der Formel (I-13)

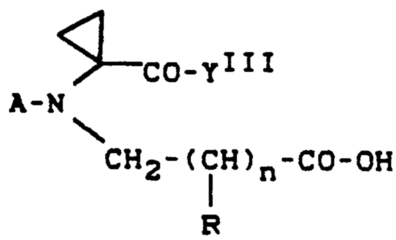

(I-13)

in welcher A, R und n die oben angegebenen Bedeutungen haben und $Y^{III}$ für die Gruppierung $NR^2R^3$ oder $OR^5$ steht, wobei $R^2$, $R^3$ und $R^5$ die oben angegebenen Bedeutungen haben,

wenn man die Benzylester der Formel (I-14)

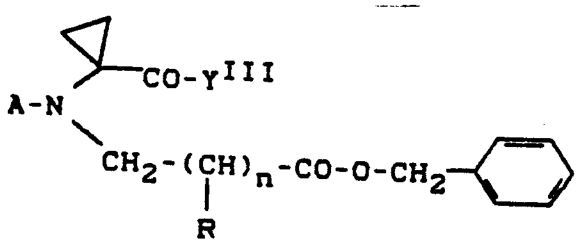

(I-14)

in welcher A, $Y^{III}$, R, und n die oben angegebenen Bedeutungen haben, mit Wasserstoff in Gegenwart eines Katalysators und und in Gegenwart eines Lösungsmittels hydriert, oder man erhält

i) die erfindungsgemäßen substituierten 1-Amino-cyclopropancarbonsäurederivate der Formel (I-15)

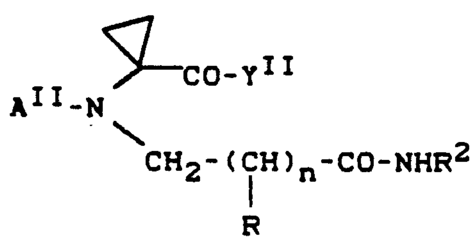

(I-15)

in welcher $A^{II}$, $Y^{II}$, R, n und $R^2$ die oben angegebenen Bedeutungen haben, wenn man die erfindungsgemäßen Ester der Formel (I-12)

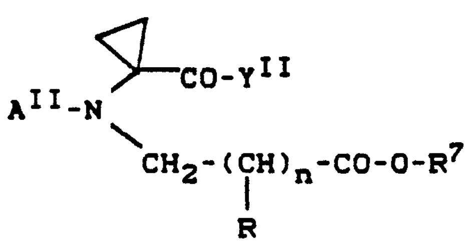

(I-12)

in welcher $A^{II}$, $Y^{II}$, R, n und $R^7$ die oben angegebenen Bedeutungen haben, mit Aminen der Formel (VI)

$H_2NR^2$ (VI)

in welcher $R^2$ die oben angegebene Bedeutung hat, in Gegenwart eines Lösungsmittels umsetzt, oder man erhält

k) die erfindungsgemäßen substituierten 1-Amino-cyclopropancarbonsäurederivate der Formel (I-16)

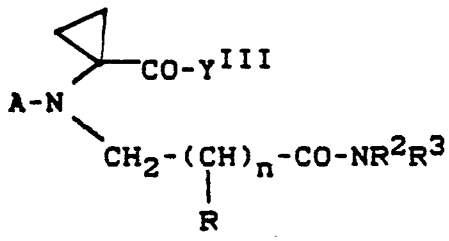

(I-16)

in welcher A, $Y^{III}$, R, n, $R^2$ und $R^3$ die oben angegebenen Bedeutungen haben, wenn man carboxy-aktivierte Derivate der erfindungsgemäßen Carbonsäuren der Formel (I-13)

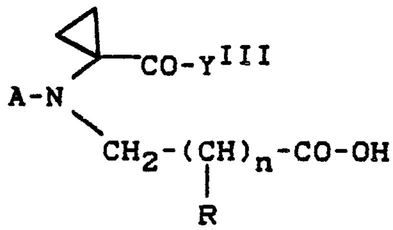

(I-13)

in welcher A, $Y^{III}$, R und n die oben angegebenen Bedeutungen haben, mit Aminen der Formel (VII)

$HNR^2R^3$ (VII)

in welcher $R^2$ und $R^3$ die oben angegebenen Bedeutungen haben, gegebenenfalls in Gegenwart einer Base, gegebenenfalls in Gegenwart eines Lösungsmittels und gegebenenfalls in Gegenwart eines Katalysators umsetzt, oder man erhält

l) die erfindungsgemäßen substituierten 1-Amino-cyclopropancarbonsäurederivate der Formel (I-17)

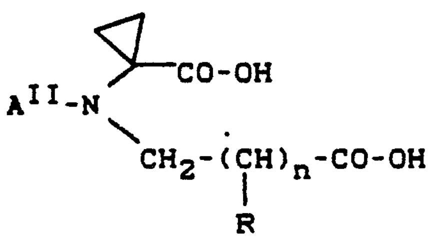

(I-17)

in welcher $A^{II}$, R und n die oben angegebenen Bedeutungen haben, wenn man die erfindungsgemäßen Diester der Formel (I-18)

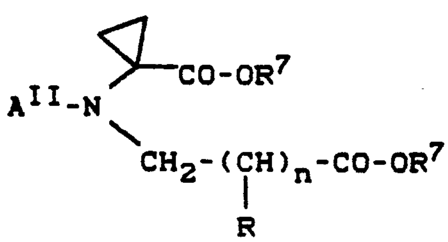

(I-18)

in Gegenwart einer wäßrigen Base und gegebenenfalls in Gegenwart eines Verdünnungsmittels hydrolisiert, oder man erhält

m) die erfindungsgemäßen substituierten 1-Amino-cyclopropancarbonsäurederivate der Formel (I-19)

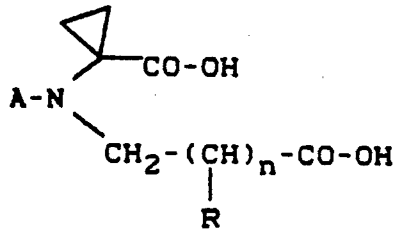

(I-19)

in welcher A, R und n die oben angegebenen Bedeutungen haben,
wenn man die Dibenzylester der Formel (I-20)

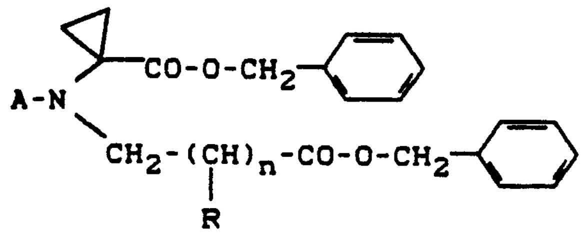

(I-20)

in welcher A, R und n die oben angegebenen Bedeutungen haben,
mit Wasserstoff in Gegenwart eines Katalysators und in Gegenwart eines Lösungsmittels hydriert,
oder man erhält

n) die erfindungsgemäßen substituierten 1-Amino-cyclopropancarbonsäurederivate der Formel (I-21)

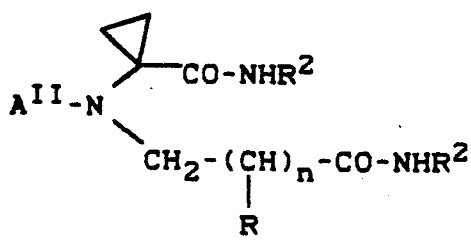

(I-21)

in welcher $A^{II}$, $R^2$, R und n die oben angegebenen Bedeutungen haben,
wenn man die erfindungsgemäßen Diester der Formel (I-18)

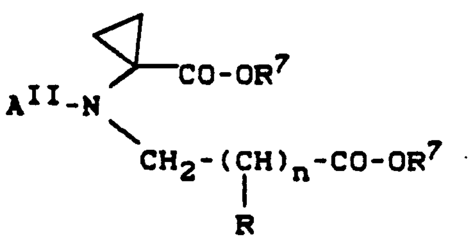

(I-18)

in welcher $A^{II}$, $R^7$, R und n die oben angegebenen Bedeutungen haben,
mit Aminen der Formel (VI)
$H_2NR^2$ (VI)
in welcher $R^2$ die oben angegebene Bedeutung hat,
in Gegenwart eines Lösungsmittels umsetzt,
oder man erhält

o) die erfindungsgemäßen substituierten 1-Amino-cyclopropancarbonsäurederivate der Formel (I-22)

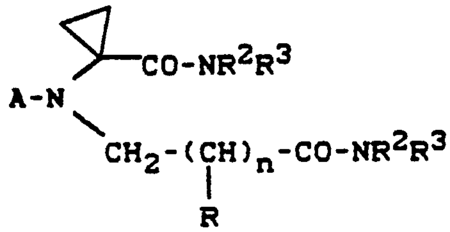

(I-22)

in welcher A, $R^2$, $R^3$, R und n die oben angegebenen Bedeutungen haben, wenn man carboxy-aktivierte Derivate der erfindungsgemäßen Dicarbonsäuren der Formel (I-19)

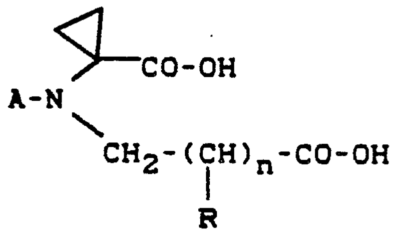

(I-19)

in welcher A, R und n die oben angegebenen Bedeutungen haben, mit Aminen der Formel (VII)

$HNR^2R^3$ (VII)

in welcher $R^2$ und $R^3$ die oben angegebenen Bedeutungen haben, gegebenenfalls in Gegenwart einer Base, in Gegenwart eines Lösungsmittels und gegebenenfalls in Gegenwart eines Katalysators umsetzt,

oder man erhält

p) die erfindungsgemäßen substituierten 1-Amino-cyclopropancarbonsäurederivate der Formel (I-23)

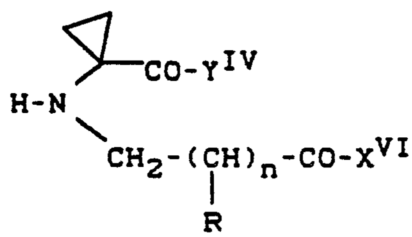

(I-23)

in welcher

R und n die oben angegebenen Bedeutungen haben,

$Y^{IV}$ für die Bedeutung von Y mit Ausnahme von tert.-Alkoxy steht, und

$X^{VI}$ für die Bedeutungen von X mit Ausnahme von tert.-Alkoxycarbonyl steht,

wenn man die erfindungsgemäßen Formylderivate der Formel (I-24)

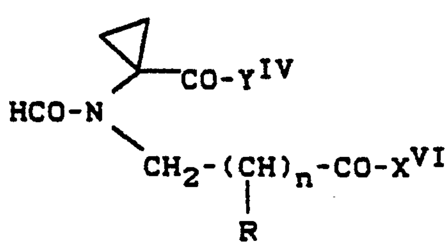

(I-24)

in welcher $Y^{IV}$, R, n und $X^{VI}$ die oben angegebenen Bedeutungen haben, mit einer Säure in Gegenwart eines Lösungsmittels behandelt,

oder man erhält

q) die erfindungsgemäßen substituierten 1-Amino-cyclopropancarbonsäurederivate der Formel (I-25)

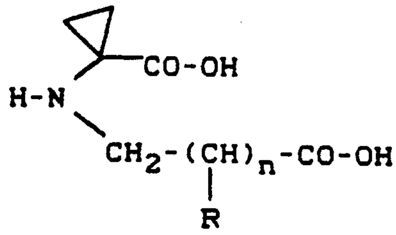

(I-25)

in welcher
R und n die oben angegebenen Bedeutungen haben,
wenn man die erfindungsgemäßen Formylderivate der Formel (I-26)

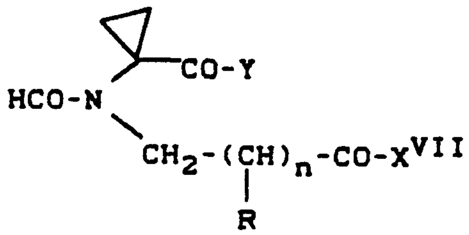

(I-26)

in welcher Y, R, und n die oben angegebenen Bedeutungen haben und
$X^{VII}$ für Hydroxycarbonyl, Alkoxycarbonyl, Aminocarbonyl, Alkylaminocarbonyl, Dialkylaminocarbonyl oder Cyano steht,
mit wäßrigen Mineralsäuren gegebenenfalls in Gegenwart eines Lösungsmittels erhitzt,
oder man erhält

r) die erfindungsgemäßen substituierten 1-Amino-cyclopropancarbonsäurederivate der Formel (I-27)

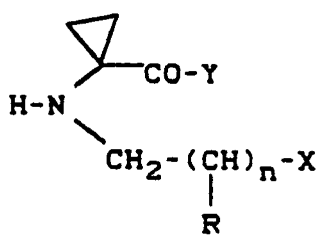

(I-27)

in welcher Y, R, n und X die oben angegebenen Bedeutungen haben,
wenn man die Trifluoracetylderivate der Formel (I-28)

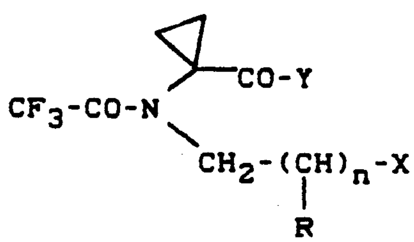

(I-28)

mit einer BAse in Gegenwart eines Verdünnungsmittels umsetzt,
oder man erhält

s) die erfindungsgemäßen substituierten 1-Amino-cyclopropancarbonsäurederivate der Formel (I-29)

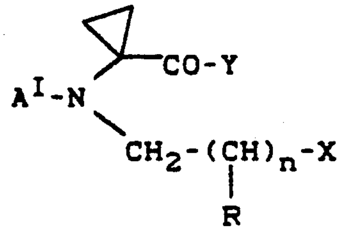

(I-29)

in welcher $A^I$, Y, R, n und X die oben angegebenen Bedeutungen haben, wenn man die erfindungsgemäßen Verbindungen der Formel (I-27)

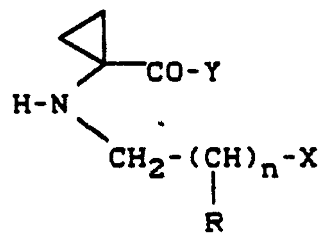

(I-27)

in welcher Y, R, n und X die oben angegebenen Bedeutungen haben, mit carboxy-aktivierten Derivaten der Carbonsäuren der Formel (VIII)

$A^I$-OH (VIII)

in welcher $A^I$ die oben angegebene Bedeutung hat, gegebenenfalls in Gegenwart einer Base, in Gegenwart eines Lösungsmitels und gegebenenfalls in Gegenwart eines Katalysators acyliert.

Die so zu erhaltenen Verbindungen der Formel (I), in denen Y für eine Hydroxy-Gruppe steht, können mit Aminen und Alkali-bzw. Erdalkalihydroxiden, -carbonaten oder -hydrogencarbonaten sowie Ammoniumhydroxid Salze bilden.

Ebenso können von den Verbindungen der Formel (I) gegebenenfalls Säureadditions-Salze hergestellt werden.

Alkyl bedeutet in der Definition von $R^1$ erfindungsgemäß geradkettiges oder verzweigtes Alkyl mit 1 bis 10 Kohlenstoffatomen, vorzugsweise mit 1 bis 8 Kohlenstoffatomen. Genannt seien Methyl, Ethyl, n-Propyl, iso-Propyl, n-Butyl, s-Butyl, i-Butyl, t-Butyl, n-Pentyl, n-Hexyl, n-Heptyl, n-Octyl, n-Nonyl, n-Decyl, i-Pentyl und neo-Pentyl.

Alkyl bedeutet in den Definitionen von $R^2$, $R^3$ und W erfindungsgemäß geradkettiges oder verzweigtes Alkyl mit 1 bis 6, vorzugsweise 1 bis 4, Kohlenstoffatomen.

Alkyl bedeutet in den Definitionen von R erfindungsgemäß geradkettiges oder verzweigtes Alkyl mit 1 bis 4, vorzugsweise 1 bis 3, Kohlenstoffatomen.

Alkyl bedeutet in $R^1$ als Benzylsubstituent erfindungsgemäß geradkettiges oder verzweigtes Alkyl mit 1 bis 4, vorzugsweise 1 bis 3, Kohlenstoffatomen.

Alkenyl und Alkinyl bedeuten unabhängig voneinander in den Definitionen von W und X erfindungsgemäß jeweils Alkenyl und Alkinyl mit je 2 bis 4, vorzugsweise 2, Kohlenstoffatomen. Es seien genannt Vinyl, Propenyl, Butenyl, Ethinyl, Propargyl und Butinyl.

Halogenalkyl bedeutet in den Definitionen in $R^1$ als Benzylsubstituent und in W erfindungsgemäß jeweils Halogenalkyl mit 1 oder 2 Kohlenstoffatomen und 1 bis 5 gleichen oder verschiedenen Halogenatomen. Genannt seien Trifluormethyl, Chlormethyl, Dichlormethyl, Trichlormethyl, Dichlorethyl und Trichlorethyl.

Alkoxycarbonyl bedeutet in den Definitionen von W und X erfindungsgemäß Alkoxycarbonyl mit 1 bis 4, vorzugsweise 1 oder 2, Kohlenstoffatomen im Alkoxyteil.

Alkoxyalkyl bedeutet in der Definition von W erfindungsgemäß geradkettiges oder verzweigtes Alkoxyalkyl mit 1 bis 4, vorzugsweise 1 oder 2, Kohlenstoffatomen je Alkoxy-und Alkylteil.

Cycloalkyl bedeutet in der Definition von W erfindungsgemäß Cycloalkyl mit 3 bis 6 Kohlenstoffatomen. Genannt seien Cyclopropyl, Cyclobutyl, Cyclopentyl und Cyclohexyl.

Alkylaminocarbonyl und Dialkylaminocarbonyl bedeuten in der Definition von X jeweils geradkettiges oder verzweigtes Alkyl mit 1 bis 4, vorzugsweise 1 oder 2, Kohlenstoffatomen je Alkylrest.

Schließlich wurde gefunden, daß die neuen substituierten 1-Amino-cyclopropancarbonsäurederivate der Formel (I) sowie deren pflanzenverträglichen Säureadditionssalze und gegebenenfalls substituierten Ammonium-, Alkali-und Erdalkali-Salze gegen Schädlinge, besonders gegen Pilze, eingesetzt werden können. Dabei zeigen überraschenderweise die erfindungsgemäßen Verbindungen eine höhere Wirkung als

die aus dem Stand der Technik bekannten Verbindungen, wie beispielsweise 1-Methylamino-cyclopropancarbonsäure, 1-(N-Formyl-N-methylamino)-cyclopropancarbonsäure und 1-(N-Formyl-N-methylamino)-cyclopropancarbonsäuremethylester, welche konstitutionell naheliegende Verbindungen sind. Die erfindungsgemäßen Stoffe stellen somit eine Bereicherung der Technik dar.

Die erfindungsgemäßen substituierten 1-Amino-cyclopropancarbonsäurederivate sind durch die Formel (I) allgemein definiert. Bevorzugt sind Verbindungen der Formel (I), in denen

Y für die Gruppierung $OR^1$ oder $-NR^2R^3$ steht, wobei

$R^1$ für Wasserstoff, für geradkettiges oder verzweigtes Alkyl mit 1 bis 10 Kohlenstoffatomen oder für gegebenenfalls ein-bis fünffach, gleich oder verschieden substituiertes Benzyl steht, wobei als Substituenten Halogen, Alkyl mit 1 bis 4 Kohlenstoffatomen und Halogenalkyl mit 1 oder 2 Kohlenstoffatomen und 1 bis 5 gleichen oder verschiedenen Halogenatomen genannt seien,

$R^2$ für Wasserstoff oder für geradkettiges oder verzweigtes Alkyl mit 1 bis 6 Kohlenstoffatomen steht, und

$R^3$ für Wasserstoff oder für geradkettiges oder verzweigtes Alkyl mit 1 bis 6 Kohlenstoffatomen steht, oder

$R^2$ und $R^3$ gemeinsam mit dem Stickstoffatom, an das sie gebunden sind, für einen gegebenenfalls substituierten 5-bis 7-gliedrigen Ring stehen, der weitere gleiche oder verschiedene Heteroatome, wie Sauerstoff oder Stickstoff, enthalten kann,

A für Wasserstoff oder für den Rest W-CO-steht, wobei

W für Wasserstoff, für geradkettiges oder verzweigtes Alkyl mit 1 bis 6 Kohlenstoffatomen, für Alkenyl oder Alkinyl mit 2 bis 4 Kohlenstoffatomen, für Cycloalkyl mit 3 bis 6 Kohlenstoffatomen, für Halogenalkyl mit 1 oder 2 Kohlenstoffatomen und 1 bis 5 gleichen oder verschiedenen Halogenatomen, wie Fluor-oder Chloratomen, für Alkoxyalkyl oder Alkoxycarbonyl mit jeweils 1 bis 4 Kohlenstoffatomen in jedem Alkylteil bzw. Alkoxyteil, für den Rest $CH_3SO_m$-$CH_2$-steht, wobei

m für die Zahlen 0, 1 oder 2 steht,

für Phenyl, Benzyl oder für einen 5-oder 6-gliedrigen heterocyclischen Ring mit 1 oder 2 weiteren, gleichen oder verschiedenen Heteroatomen, wie Sauerstoff und Stickstoff, steht,

R für Wasserstoff oder geradkettiges oder verzweigtes Alkyl mit 1 bis 4 Kohlenstoffatomen steht,

n für die Zahlen 0, 1 oder 2 steht, und

X für Alkenyl oder Alkinyl mit 2 bis 4 Kohlenstoffatomen, für Hydroxycarbonyl, für Alkoxycarbonyl mit 1 bis 4 Kohlenstoffatomen im Alkoxyteil, für Aminocarbonyl, für Alkylaminocarbonyl oder Dialkylaminocarbonyl mit jeweils 1 bis 4 Kohlenstoffatomen in jedem Alkylteil oder für Cyano steht.

Besonders bevorzugt sind diejenigen Verbindungen der Formel (I), in denen

Y für die Gruppierung $OR^1$ oder $NR^2R^3$ steht, in welchen

$R^1$ für Wasserstoff, für geradkettiges oder verzweigtes Alkyl mit 1 bis 8 Kohlenstoffatomen oder für gegebenenfalls ein-bis dreifach, gleich oder verschieden substituiertes Benzyl steht, wobei als Substituenten Fluor, Chlor, Brom, Methyl, Ethyl, n-und i-Propyl und Trifluormethyl genannt seien,

$R^2$ für Wasserstoff, Methyl, Ethyl, n-und i-Propyl oder n-, i-, s-und t-Butyl steht, und

$R^3$ für Wasserstoff, Methyl, Ethyl, n-und i-Propyl oder n-, i-, s-und t-Butyl steht, oder

$R^2$ und $R^3$ gemeinsam mit dem Stickstoffatom, an das sie gebunden sind, für Pyrrolidin, Piperidin, Morpholin oder N-Methylpiperazin stehen,

A für Wasserstoff oder für den Rest W-CO-steht, wobei

W für Wasserstoff, Methyl, Ethyl, n-und i-Propyl, n-, i-, s-und t-Butyl, Vinyl, Ethinyl, Cyclopropyl, Cyclopentyl, Cyclohexyl, Trifluormethyl, Chlormethyl, Dichlormethyl, Trichlormethyl, Methoxymethyl, 2-Methoxyethyl, 2-Ethoxyethyl, Methoxycarbonyl, Ethoxycarbonyl, Methylthiomethyl, Methylsulfinylmethyl, Methylsulfonylmethyl, Phenyl, Benzyl, Furan-2-yl oder Isoxazol-5-yl steht,

R für Wasserstoff, Methyl, Ethyl oder n-und i-Propyl steht,

n für die Zahlen 0, 1 oder 2 steht, und

X für Vinyl, Ethinyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht.

Insbesondere seien Verbindungen der Formel I genannt, in denen

Y für die Gruppierungen $OR^1$ oder $NR^2R^3$ steht, in welchen

$R^1$ für Wasserstoff, für geradkettiges oder verzweigtes Alkyl mit 1 bis 8 Kohlenstoffatomen oder für gegebenenfalls ein-bis dreifach, gleich oder verschieden durch Fluor, Chlor, Brom, Methyl, Ethyl, n-Propyl, iso-Propyl und Trifluormethyl substituiertes Benzyl steht,

$R^2$ für Wasserstoff, Methyl, Ethyl, n-Propyl, iso-Propyl, n-, i-, s-oder t-Butyl steht

$R^3$ für Wasserstoff, Methyl, Ethyl, n-und i-Propyl oder n-, i-, s-und t-Butyl steht oder

$R^2$ und $R^3$ gemeinsam mit dem Stickstoffatom, an das sie gebunden sind, für Pyrrolidin, Piperidin, Morpholin oder N-Methylpiperazin stehen,

A für Wasserstoff oder den Rest HCO-steht,

R für Wasserstoff, Methyl, Ethyl oder n-und i-Propyl steht,
n für die Zahlen 0, 1 oder 2 steht und
X für Vinyl, Ethinyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht.

Die Ausgangs-bzw. Endprodukte in den Verfahren a) bis s) sind durch die Formeln (I-1) bis (I-29) und - (II) bis (VIII) allgemein definiert.

Bevorzugt sind Verbindungen der Formeln (I-1) bis (I-29) und (II) bis (VIII), in denen
Y für die Gruppierung $OR^1$ oder $-NR^2R^3$ steht, wobei
$R^1$ für Wasserstoff, für geradkettiges oder verzweigtes Alkyl mit 1 bis 10 Kohlenstoffatomen oder für gegebenenfalls ein-bis fünffach, gleich oder verschieden substituiertes Benzyl steht, wobei als Substituenten Halogen, Alkyl mit 1 bis 4 Kohlenstoffatomen und Halogenalkyl mit 1 oder 2 Kohlenstoffatomen und 1 bis 5 gleichen oder verschiedenen Halogenatomen genannt seien,
$R^2$ für Wasserstoff oder für geradkettiges oder verzweigtes Alkyl mit 1 bis 6 Kohlenstoffatomen steht,
$R^3$ für Wasserstoff oder für geradkettiges oder verzweigtes Alkyl mit 1 bis 6 Kohlenstoffatomen steht oder
$R^2$ und $R^3$ gemeinsam mit dem Stickstoffatom, an das sie gebunden sind, für einen gegebenenfalls substituierten 5-bis 7-gliedrigen Ring stehen, der weitere gleiche oder verschiedene Heteroatome, wie Sauerstoff oder Stickstoff, enthalten kann,
$Y^I$ für die Gruppierung $OR^4$ und $NR^5R^6$ steht, wobei
$R^4$ für geradkettiges oder verzweigtes Alkyl mit 1 bis 10 Kohlenstoffatomen oder für gegebenenfalls ein-bis fünffach, gleich oder verschieden substituiertes Benzyl steht, wobei als Substituenten Halogen, Alkyl mit 1 bis 4 Kohlenstoffatomen und Halogenalkyl mit 1 oder 2 Kohlenstoffatomen und 1 bis 5 gleichen oder verschiedenen Halogenatomen genannt seien,
$R^5$ für geradkettiges oder verzweigtes Alkyl mit 1 bis 6 Kohlenstoffatomen steht,
$R^6$ für geradkettiges oder verzweigtes Alkyl mit 1 bis 6 Kohlenstoffatomen steht oder
$R^5$ und $R^6$ gemeinsam mit dem Stickstoffatom, an das sie gebunden sind, für einen gegebenenfalls substituierten 5-bis 7-gliedrigen Ring stehen, der weitere gleiche oder verschiedene Heteroatome, wie Sauerstoff oder Stickstoff, enthalten kann,
$Y^{II}$ für tert.-Alkoxy oder die Gruppierung $NR^2R^3$ steht, wobei
$R^2$ und $R^3$ vorzugsweise die oben angegebenen Bedeutungen haben,
$Y^{III}$ für die Gruppierungen $NR^2R^3$ oder $OR^5$ steht, wobei
$R^2$, $R^3$ und $R^5$ vorzugsweise die oben angegebenen Bedeutungen haben,
$Y^{IV}$ für die Gruppierung $OR^8$ und $-NR^2R^3$ steht, wobei
$R^8$ für Wasserstoff, für primäres oder sekundäres Alkyl mit 1 bis 10 Kohlenstoffatomen oder für gegebenenfalls ein-bis fünffach, gleich oder verschieden substituiertes Benzyl steht, wobei als Substituenten Halogen, Alkyl mit 1 bis 4 Kohlenstoffatomen und Halogenalkyl mit 1 oder 2 Kohlenstoffatomen und 1 bis 5 gleichen oder verschiedenen Halogenatomen genannt seien, und
$R^2$ und $R^3$ vorzugsweise die oben angegebenen Bedeutungen haben,
A für Wasserstoff oder für den Rest W-CO-steht, wobei
W für Wasserstoff, für geradkettiges oder verzweigtes Alkyl mit 1 bis 6 Kohlenstoffatomen, für Alkenyl oder Alkinyl mit 2 bis 4 Kohlenstoffatomen, für Cycloalkyl mit 3 bis 6 Kohlenstoffatomen, für Halogenalkyl mit 1 oder 2 Kohlenstoffatomen und 1 bis 5 gleichen oder verschiedenen Halogenatomen, wie Fluor-oder Chloratomen, für Alkoxyalkyl oder Alkoxycarbonyl mit jeweils 1 bis 4 Kohlenstoffatomen in jedem Alkylteil bzw. Alkoxyteil, für den Rest $CH_3SO_m-CH_2-$, wobei
m für die Zahlen 0, 1 oder 2 steht,
oder für Phenyl, Benzyl oder für einen 5-oder 6-gliedrigen heterocyclischen Ring mit 1 oder 2 gleichen oder verschiedenen Heteroatomen, wie Sauerstoff oder Stickstoff, steht,
$A^I$ für den Rest W-CO-steht, wobei
W für Wasserstoff, für geradkettiges oder verzweigtes Alkyl mit 1 bis 6 Kohlenstoffatomen, für Alkenyl oder Alkinyl mit 2 bis 4 Kohlenstoffatomen, für Cycloalkyl mit 3 bis 6 Kohlenstoffatomen, für Halogenalkyl mit 1 oder 2 Kohlenstoffatomen und 1 bis 5 gleichen oder verschiedenen Halogenatomen, wie Fluor-oder Chloratom, für Alkoxyalkyl oder Alkoxycarbonyl mit jeweils 1 bis 4 Kohlenstoffatomen in jedem Alkylteil, für den Rest $CH_3SO_m-CH_2-$, wobei
m für die Zahlen 0, 1 oder 2 steht;
oder für Phenyl, Benzyl oder für einen 5-oder 6-gliedrigen heterocyclischen Ring mit 1 oder 2 gleichen oder verschiedenen Heteroatomen, wie Sauerstoff oder Stickstoff, steht,
$A^{II}$ für Wasserstoff oder für den Rest $W^I$-CO-steht, wobei
$W^I$ für Wasserstoff, für geradkettiges oder verzweigtes Alkyl mit 1 bis 6 Kohlenstoffatomen, für Alkenyl oder Alkinyl mit 2 bis 4 Kohlenstoffatomen, für Cycloalkyl mit 3 bis 6 Kohlenstoffatomen, für Alkoxyalkyl oder

Alkoxycarbonyl mit jeweils 1 bis 4 Kohlenstoffatomen in jedem Alkylteil bzw. Alkoxyteil, für den Rest $CH_3$-SO $_m$-$CH_2$-, wobei
m für die Zahlen 0, 1 oder 2 steht,
für Phenyl, Benzyl oder für einen 5-oder 6-gliedrigen heterocyclischen Ring mit 1 oder 2 gleichen oder verschiedenen Heteroatomen, wie Sauerstoff oder Stickstoff, steht,
R für Wasserstoff oder geradkettiges oder verzweigtes Alkyl mit 1 bis 4 Kohlenstoffatomen steht,
n für die Zahlen 0, 1 oder 2 steht,
X für Alkenyl oder Alkinyl mit 2 bis 4 Kohlenstoffatomen, für Hydroxycarbonyl, für Alkoxycarbonyl mit 1 bis 4 Kohlenstoffatomen im Alkoxyteil, für Aminocarbonyl, für Alkylaminocarbonyl oder Dialkylaminocarbonyl mit jeweils 1 bis 4 Kohlenstoffatomen in jedem Alkylteil oder für Cyano steht,
$X^I$ für Alkenyl oder Alkinyl mit 2 bis 4 Kohlenstoffatomen, für Alkoxycarbonyl mit 1 bis 4 Kohlenstoffatomen im Alkoxyteil oder Dialkylaminocarbonyl mit 1 bis 4 Kohlenstoffatomen in jedem Alkylteil oder für Cyano steht,
$X^{II}$ für Alkoxycarbonyl mit 1 bis 4 Kohlenstoffatomen im Alkoxyteil oder für Cyano steht,
$X^{III}$ für Alkenyl oder Alkinyl mit 2 bis 4 Kohlenstoffatomen, für Hydroxycarbonyl, für tert.-Butoxycarbonyl, für Aminocarbonyl, für Alkylaminocarbonyl oder Dialkylaminocarbonyl mit jeweils 1 bis 4 Kohlenstoffatomen in jedem Alkylteil oder für Cyano steht,
$X^{IV}$ für Alkenyl oder Alkinyl mit 2 bis 4 Kohlenstoffatomen, für Hydroxycarbonyl, für i-Propoxycarbonyl oder i-, s-und t-Butoxycarbonyl, für Aminocarbonyl, für Alkylaminocarbonyl oder Dialkylaminocarbonyl mit jeweils 1 bis 4 Kohlenstoffatomen in jedem Alkylteil oder für Cyano steht,
$X^V$ für Alkenyl oder Alkinyl mit 2 bis 4 Kohlenstoffatomen, für Alkoxycarbonyl mit 1 bis 4 Kohlenstoffatomen im Alkoxyteil, für Aminocarbonyl, für Alkylaminocarbonyl oder Dialkylaminocarbonyl mit jeweils 1 bis 4 Kohlenstoffatomen in jedem Alkylteil oder für Cyano steht,
$X^{VI}$ für Alkenyl oder Alkinyl mit 2 bis 4 Kohlenstoffatomen, für Hydroxycarbonyl, für primäres oder sekundäres Alkoxycarbonyl mit 1 bis 4 Kohlenstoffatomen im Alkoxyteil, für Aminocarbonyl, für Alkylaminocarbonyl oder Dialkylaminocarbonyl mit jeweils 1 bis 4 Kohlenstoffatomen in jedem Alkylteil oder Cyano steht,
$X^{VII}$für Hydroxycarbonyl, für Alkoxycarbonyl mit 1 bis 4 Kohlenstoffatomen im Alkoxyteil, für Aminocarbonyl, für Alkylaminocarbonyl oder Dialkylaminocarbonyl mit jeweils 1 bis 4 Kohlenstoffatomen in jedem Alkylteil oder für Cyano steht,
$R^7$ für Methyl oder Ethyl steht und
Z für Chlor, Brom, Jod, Mesyloxy oder Tosyloxy steht.

Besonders bevorzugt sind diejenigen Verbindungen der Formel (I-1) bis (I-29) und (II) bis (VIII), in denen
Y für die Gruppierung $OR^1$ und $NR^2R^3$ steht, wobei
$R^1$ für Wasserstoff, für geradkettiges oder verzweigtes Alkyl mit 1 bis 8 Kohlenstoffatomen oder für gegebenenfalls ein bis fünffach, gleich oder verschieden substituiertes Benzyl steht, wobei als Substituenten Fluor, Chlor, Brom, Methyl, Ethyl, n-und i-Propyl und Trifluormethyl genannt seien,
$R^2$ für Wasserstoff, Methyl, Ethyl, n-und i-Propyl oder n-, i-, s-und t-Butyl steht,
$R^3$ für Wasserstoff, Methyl, Ethyl, n-und i-Propyl oder n-, i-, s-und t-Butyl steht oder
$R^2$ und $R^3$ gemeinsam mit dem Stickstoffatom, an das sie gebunden sind, für Pyrrolidin, Piperidin, Morpholin oder N-Methylpiperazin stehen.
$Y^I$ für die Gruppierung $OR^4$ oder $NR^5R^6$ steht, wobei
$R^4$ für geradkettiges oder verzweigtes Alkyl mit 1 bis 8 Kohlenstoffatomen oder für gegebenenfalls ein bis dreifach, gleich oder verschieden substituiertes Benzyl steht, wobei als Substituenten Fluor, Chlor, Brom, Methyl, Ethyl, n-und i-Propyl und Trifluormethyl genannt seien,
$R^5$ für Wasserstoff, Methyl, Ethyl, n-und i-Propyl oder n-, i-, s-und t-Butyl steht,
$R^6$ für Wasserstoff, Methyl, Ethyl, n-und i-Propyl oder n-, i-, s-und t-Butyl steht oder
$R^5$ und $R^6$ gemeinsam mit dem Stickstoffatom, an das sie gebunden sind, für Pyrrolidin, Piperidin, Morpholin oder N-Methylpiperazin stehen.
$Y^{II}$ für tert.-Alkoxy oder die Gruppierung $NR^2R^3$ steht, wobei
$R^2$ und $R^3$ die oben angegebenen Bedeutungen haben,
$Y^{III}$ für die Gruppierung $NR^2R^3$ oder $OR^5$ steht, wobei
$R^2$, $R^3$ und $R^5$ die oben angegebenen Bedeutungen haben,
$Y^{IV}$ für die Gruppierung $OR^8$ oder $NR^2R^3$ steht, wobei
$R^8$ für Wasserstoff, für primäres oder sekundäres Alkyl mit 1 bis 8 Kohlenstoffatomen oder für gegebenenfalls ein-bis dreifach, gleich oder verschieden substituiertes Benzyl steht, wobei als Substituenten Fluor, Chlor, Brom, Methyl, Ethyl, n-und i-Propyl und Trifluormethyl genannt seien, und

$R^2$ und $R^3$ die oben angegebenen Bedeutungen haben,
A für Wasserstoff oder für den Rest W-CO-steht, wobei
W für Wasserstoff, Methyl, Ethyl, n-und i-Propyl, n-, i-, s-und t-Butyl, Vinyl, Ethinyl, Cyclopropyl, Cyclopentyl, Cyclohexyl, Trifluormethyl, Chlormethyl, Dichlormethyl, Trichlormethyl, Methoxymethyl, 2-Methoxyethyl, 2-Ethoxyethyl, Methoxycarbonyl, Ethoxycarbonyl, Methylthiomethyl, Methylsulfinylmethyl, Methylsulfonylmethyl, Phenyl, Benzyl, Furan-2-yl oder Isoxazol-5-yl steht,
$A^I$ für den Rest W-CO-steht, wobei
W für Wasserstoff, Methyl, Ethyl, n-und i-Propyl, n-, i-, s-und t-Butyl, Vinyl, Ethinyl, Cyclopropyl, Cyclopentyl, Cyclohexyl, Trifluormethyl, Chlormethyl, Dichlormethyl, Trichlormethyl, Methoxymethyl, 2-Methoxyethyl, 2-Ethoxyethyl, Methoxycarbonyl, Ethoxycarbonyl, Methylthiomethyl, Methylsulfinylmethyl, Methylsulfonylmethyl, Phenyl oder Benzyl steht,
$A^{II}$ für Wasserstoff oder für den Rest $W^I$-CO-steht, wobei
$W^I$ für Wasserstoff, Methyl, Ethyl, n-und i-Propyl, n-, i-, s-und t-Butyl, Vinyl, Ethinyl, Cyclopropyl, Cyclopentyl, Cyclohexyl, Chlormethyl, Dichlormethyl, Trichlormethyl, Methoxymethyl, 2-Methoxyethyl, 2-Ethoxyethyl, Methoxycarbonyl, Ethoxycarbonyl, Methylthiomethyl, Methylsulfinylmethyl, Methylsulfonylmethyl, Phenyl oder Benzyl steht,
R für Wasserstoff, Methyl, Ethyl oder n-und i-Propyl steht,
n für die Zahlen 0, 1 oder 2 steht,
X für Vinyl, Ethinyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht.
$X^I$ für Vinyl, Ethinyl, Methoxycarbonyl, Ethoxycarbonyl, Dimethylaminocarbonyl, Diethylaminocarbonyl oder für Cyano steht,
$X^{II}$ für Methoxycarbonyl, Ethoxycarbonyl oder Cyano steht,
$X^{III}$ für Vinyl, Ethinyl, Hydroxycarbonyl, Aminocarbonyl, Methylaminocarbonyl, Ethylaminocarbonyl, Dimethylaminocarbonyl, Diethylaminocarbonyl oder für Cyano steht,
$X^{IV}$ für Vinyl, Ethinyl, Hydroxycarbonyl, Aminocarbonyl, Methylaminocarbonyl, Ethylaminocarbonyl, Dimethylaminocarbonyl, Diethylaminocarbonyl oder für Cyano steht,
$X^V$ für Vinyl, Ethinyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht,
$X^{VI}$ für Vinyl, Ethinyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht,
$X^{VII}$ für Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht,
$R^7$ für Methyl oder Ethyl steht und
Z für Chlor, Brom, Jod, Mesyloxy oder Tosyloxy steht.

Bevorzugte erfindungsgemäße Verbindungen sind auch Additionsprodukte aus Säuren und denjenigen substituierten 1-Amino-cyclopropancarbonsäurederivaten der Formel (I), in denen Y, A, R, n und X die Bedeutungen haben, die bereits für diese Substituenten und den Index n genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono-und bifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure und Milchsäure sowie Sulfonsäuren, wie z. B. p-Toluolsulfonsäure, 1,5-Naphthalindisulfonsäure und Camphersulfonsäure.

Bevorzugte erfindungsgemäße Verbindungen sind auch die Alkali-, Erdalkali-und gegebenenfalls substituierten Ammoniumsalze der substituierten 1-Amino-cyclopropancarbonsäurederivate der Formel (I), in denen Y für eine Hydroxygruppe steht und A, R, n und X die Bedeutungen haben, die bereits für diese Substituenten genannt wurden.

Für diese Salzbildung können Alkali-und Erdalkalihydroxide, -carbonate und -hydrogencarbonate oder Amine und Ammoniumhydroxid eingesetzt werden. Hierzu gehören vorzugsweise Natriumhydroxid, Natriumcarbonat, Natriumhydrogencarbonat, Kaliumhydroxid, Kaliumcarbonat, Calciumhydroxid, Bariumhydroxid; Ammoniak; primäre Amine, wie Methylamin und Isopropylamin; sekundäre Amine, wie Dimethylamin und Dicyclohexylamin; tertiäre Amine, wie Triethylamin, Pyridin und N,N-Dimethylbenzylamin sowie Ammoniumhydroxide, wie Benzyltrimethyl-ammoniumhydroxid.

Verwendet man beispielsweise 1-[N-Formyl-amino]-cyclopropancarbonsäureethylester und Chloracetonitril als Ausgangsstoffe und Kalium-tert.-butylat als Base, so kann der Ablauf des erfindungsgemäßen Verfahrens a) durch das folgende Formelschema wiedergegeben werden:

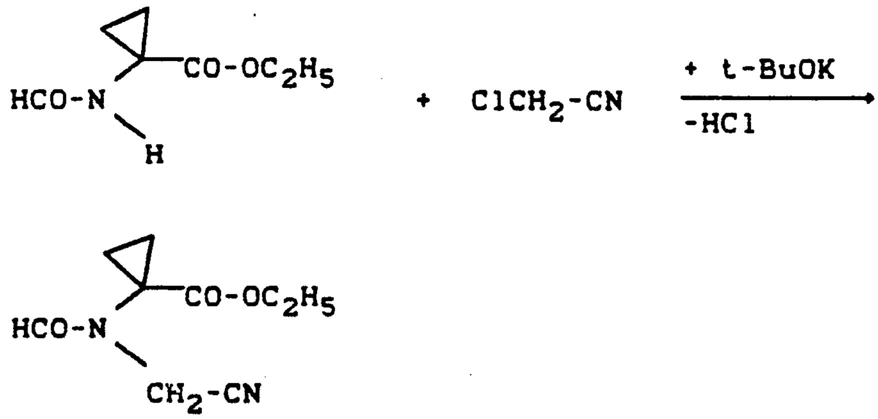


Verwendet man beispielweise 1-Aminocyclopropancarbonsäure-hydrochlorid und Acrylnitril als Ausgangsstoffe und Natronlauge als Base, so kann der Ablauf des erfindungsgemäßen Verfahrens b) durch das folgende Formelschema wiedergegeben werden:

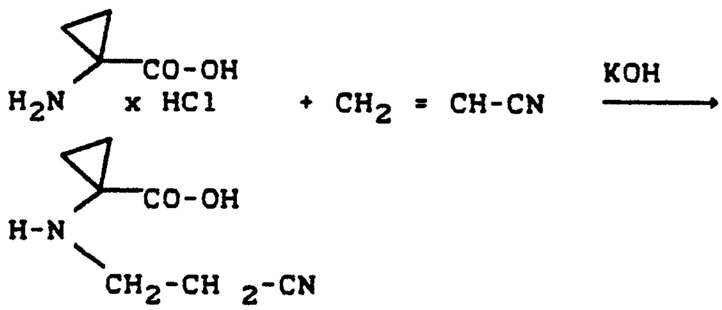


Verwendet man beispielsweise 1-[N-Cyanmethyl-N-formyl-amino]-cyclopropancarbonsäureethylester als Ausgangsstoff und Kaliumhydroxid in Wasser als wässrige Base, so kann der Ablauf des erfindungsgemäßen Verfahrens d) durch das folgende Formelschema wiedergegeben werden:

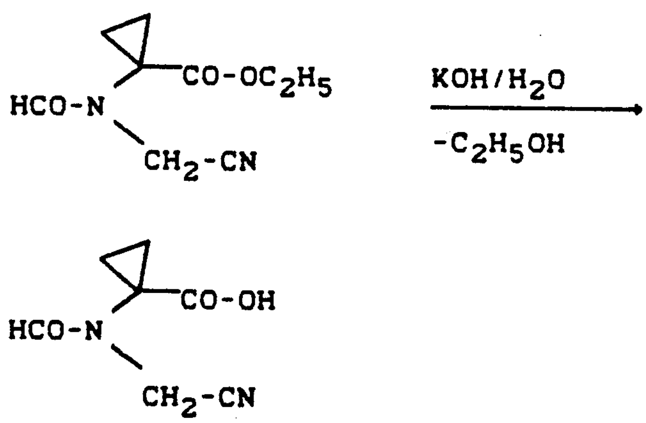


Verwendet man beispielsweise 1-[N-(2-Cyanethyl)-N-formyl-amino]-cyclopropancarbonsäureethylester und Methylamin als Ausgangsstoffe, so kann der Ablauf des erfindungsgemäßen Verfahrens e) durch das folgende Formelschema wiedergegeben werden:

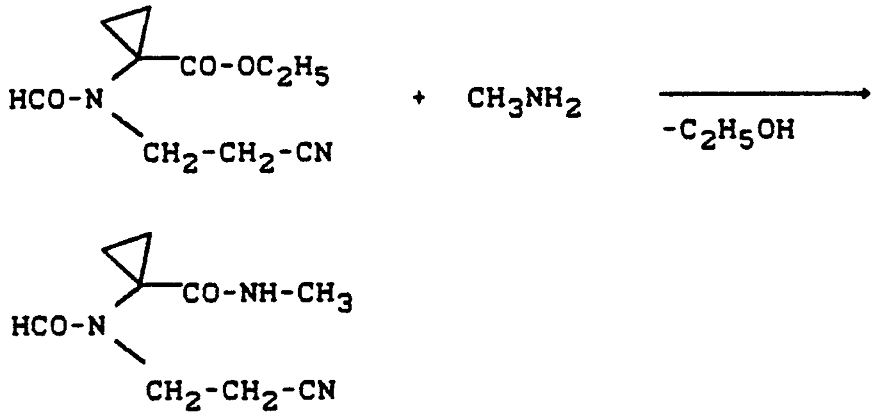


Verwendet man beispielsweise 1-[N-Ethoxycarbonylmethyl-N-formyl-amino]-cyclopropancarbonsäureethylester als Ausgangsstoff und Kaliumhydroxid in Wasser als wässrige Base, so kann der Ablauf des erfindungsgemäßen Verfahrens 1) durch das folgende Formelschema wiedergegeben werden:

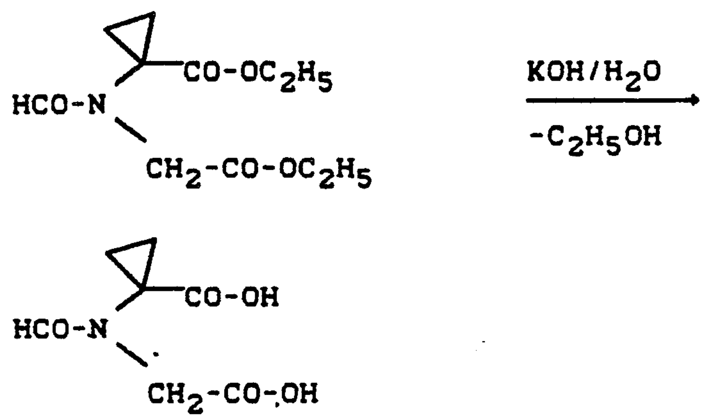


Verwendet man beispielsweise 1-[N-Ethoxycarbonylmethyl-N-formyl-amino]-cyclopropancarbonsäureethylester und Methylamin als Ausgangsstoffe, so kann der Ablauf des erfindungsgemäßen Verfahrens n) durch das folgende Formelschema wiedergegeben werden:

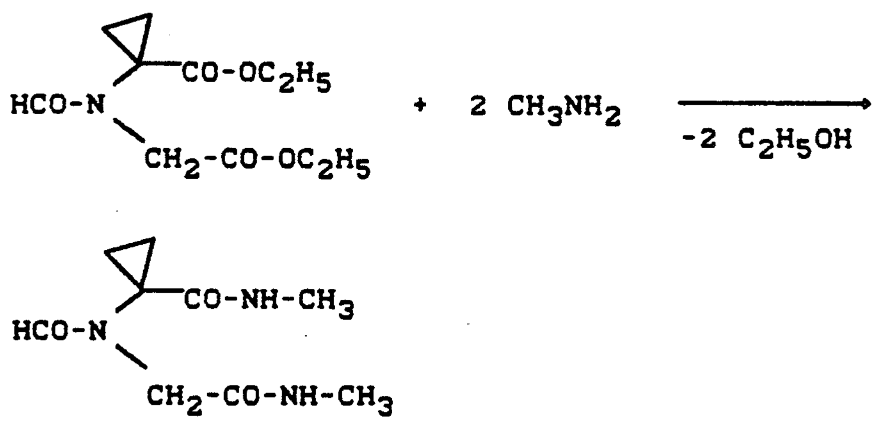


Verwendet man beispielsweise 1-[N-(2-Cyanethyl)-N-formyl-amino]-cyclopropancarbonsäureethylester als Ausgangsstoff und Salzsäure als Säure, so kann der Ablauf des erfindungsgemäßen Verfahrens p) durch das folgende Formelschema wiedergegeben werden:

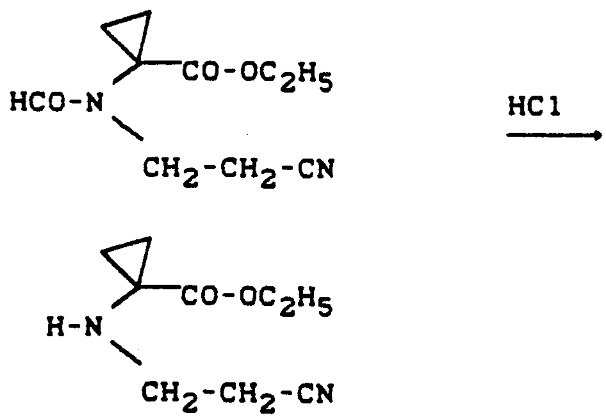


Verwendet man beispielsweise 1-[N-Ethoxycarbonylmethyl-N-formyl-amino]-cyclopropancarbonsäureethylester als Ausgangsstoff und Salzsäure als Säure, so kann der Ablauf des erfindungsgemäßen Verfahrens q) durch das folgende Formelschema wiedergegeben werden:

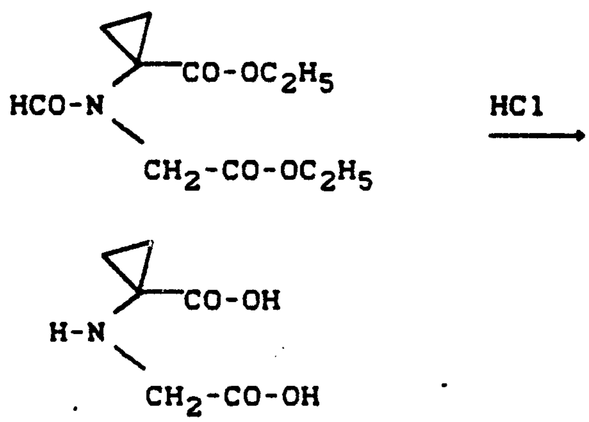


Verwendet man beispielsweise 1-[N-(2-Cyanethyl)-amino]-cyclopropancarbonsäureethylester und Propionylchlorid als Ausgangsstoffe, Triethylamin als Base und 4-Dimethylaminopyridin als Katalysator, so kann der Ablauf des erfindungsgemäßen Verfahrens s) durch das folgende Formelschema wiedergegeben werden:

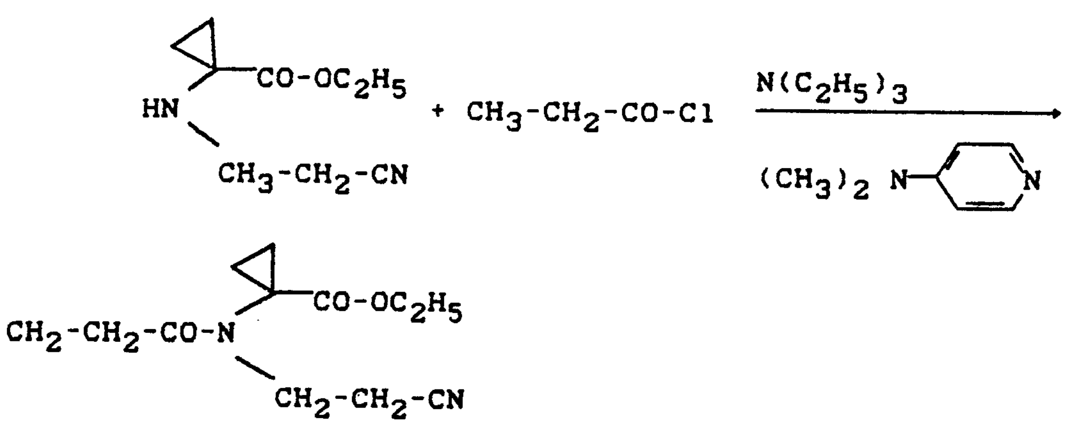


Die für die Durchführung des erfindungsgemäßen Verfahrens a) als Ausgangsstoff zu verwendenden 1-Aminocyclopropancarbonsäurederivate der Formel (II) sind teilweise bekannt (vergl. z. B. EP-OS 5 782); bzw. können sie nach im Prinzip bekannten Verfahren hergestellt werden, z. B. durch Acylierung von bekannten 1-Aminocyclopropancarbonsäureestern.

Die ebenfalls für die Durchführung des erfindungsgemäßen Verfahrens a) als Ausgangsstoffe zu verwendenden Verbindungen der Formel (III) sind allgemein bekannte Verbindungen der organischen Chemie.

Die für die Durchführung des erfindungsgemäßen Verfahrens b) als Ausgangsstoffe zu verwendenden 1-Aminocyclopropancarbonsäurederivate der Formel (IV) sind bekannt (vergl. z. B. EP-OS 5782).

Die ebenfalls für die Durchführung des erfindungsgemäßen Verfahrens b) als Ausgangsstoffe zu verwendenden Olefine der Formel (V) sind allgemein bekannte Verbindungen der organischen Chemie.

Die für die Durchführung der erfindungsgemäßen Verfahren e), i) und n) als Ausgangsstoffe zu verwendenden Amine der Formel (VI) sind allgemein bekannte Verbindungen der organischen Chemie.

Die für die Durchführung der erfindungsgemäßen Verfahren f), k) und o) als Ausgangsstoff zu verwendenden Amine der Formel VII sind allgemein bekannte Verbindungen der organischen Chemie.

Die für die Durchführung des erfindungsgemäßen Verfahrens s) als Ausgangsstoffe zu verwendenden carboxyaktivierten Derivate der Carbonsäuren der Formel (VIII) sind allgemein bekannte Verbindungen der organischen Chemie.

Als carboxyaktivierte Derivate der Carbonsäuren der Formel (VIII) kommen alle üblichen Möglichkeiten zur Aktivierung der Carboxy-Gruppe in Frage, wie insbesondere Anhydride mit anorganischen und organischen Säuren, O-Acyl-isoharnstoffe, Arylester, N-Hydroxyester und cyclische Amide (vergl. z. B. K. Lübke, E. Schröder, G. Kloss, Chemie und Biochemie der Aminosäuren, Peptide und Proteine I, S. 136, Georg Thieme Verlag Stuttgart).

Vorzugsweise werden die den Carbonsäuren der Formel (VIII) entsprechenden Säurechloride eingesetzt. Sie können hergestellt werden, indem man die Carbonsäuren der Formel (VIII) oder deren Salze mit einem Halogenierungsmittel, wie beispielsweise Phosphorpentachlorid, Thionylchlorid oder Oxalylchlorid, in allgemein bekannter Art und Weise umsetzt. Bevorzugt ist der Einsatz von Oxalylchlorid zusammen mit dem Natrium-oder Kaliumsalz der Carbonsäure der Formel (III), da unter diesen Bedingungen die Isomerisierung minimal ist.

Die Acylierung mit den carboxy-aktivierten Derivaten kann in wäßrigen oder nichtwäßrigem Medium durchgeführt werden; geeignete Medien sind dabei Ketone, wie z. B. Aceton; Ester, wie z. B. Ethylacetat; Amide, wie z. B. Dimethylformamid-, Nitrile, wie z. B. Acetonitril, Chlorkohlenwasserstoffe, wie z. B. Methylenchlorid, Kohlenwasserstoffe,
z. B. Toluol; oder Ether, wie z. B. Tetrahydrofuran oder deren Mischungen.

Die Acylierung mit einem Säurehalogenid kann in Gegenwart eines Säurebindemittels, wie vorzugsweise eines tertiären Amins, wie z. B. Triethylamin, Pyridin, oder einer anorganischen Base, wie z. B. Natriumcarbonat oder Calciumcarbonat, durchgeführt werden.

Ebenfalls werden bevorzugt die den Carbonsäuren der Formel (VIII) entsprechenden O-Alkylkohlensäureanhydride, Phosphoroxychloride, O-Acyl-isoharnstoffe, aktivierte Ester und Imidchloride eingesetzt, deren Herstellung im Zusammenhang mit der Beschreibung der carboxy-aktivierten Derivate der erfindungsgemäßen Carbonsäuren der Formeln (I-10), (I-13) und (I-19) beschrieben ist.

Die für die Durchführung der erfindungsgemäßen Verfahren c), d), e), f), g), h), i), k), l), m), n), O), p), q), r) und s) als Ausgangsstoffe zu verwendenden substituierten 1-Amino-cyclopropancarbonsäurederivate der Formeln (I-4), (I-6), (I-8), (I-10), (I-14), (I-12), (I-13), (I-18), (I-20), (I-19), (I-23), (I-26), (I-28) und (I-27) sind erfindungsgemäße Verbindungen und können gemäß den erfindungsgemäßen Verfahren hergestellt werden.

Acylierungsmittel entsprechend den Carbonsäurederivaten der Formel (I-10), (I-13) und (I-19) kommen alle carboxy-aktivierten Derivate in Frage, die im Zusammenhang mit den Acylierungsmitteln entsprechend den Carbonsäure-Derivaten der Formel (VIII) bereits genannt wurden.

Ebenfalls bevorzugt werden die den Carbonsäuren der Formel (I-10), (I-13) und (I-19) entsprechenden O-Alkylkohlensäureanhydride eingesetzt. Sie können erhalten werden, indem man 1 Äquivalent der entsprechenden Carbonsäure der Formeln (I-13), (I-10) und (I-19) und 1 Äquivalent Base, wie beispielsweise Triethylamin oder N-Methylmorpholin, in einem Lösungsmittel, wie beispielsweise Tetrahydrofuran, Dioxan, Dimethoxyethan, Acetonitril oder Pyridin, löst und bei Temperaturen zwischen -60° C und 15° C 1 Äquivalent Chlorameisensäureisobutyl-oder -ethylester zutropft und bei Temperaturen von beispielsweise - 20° C nachrührt.

Anschließend wird bei Temperaturen zwischen -60° C und 15° C möglichst schnell eine vorgekühlte Lösung des entsprechenden Amins der Formel (VII) in einem Lösungsmittel, wie beispielsweise Tetrahydrofuran, Dioxan, Dimethoxyethan, Acetonitril, Pyridin, Dimethylformamid oder Wasser zugegeben, auf Raumtemperatur erwärmt, bei Raumtemperatur nachgerührt und auf übliche Art und Weise aufgearbeitet.

Ebenfalls bevorzugt werden die den Carbonsäuren der Formeln (I-10), (I-13) und (I-19) entsprechenden Phosphoroxychloride eingesetzt. Sie können erhalten werden, indem man 1 Äquivalent der entsprechenden Carbonsäure der Formeln (I-10), (I-13) oder (I-19) und 1 Äquivalent des entsprechenden Amins der Formel - (VII) in einem inerten organischen Lösungsmittel, wie beispielsweise Pyridin, Tetrahydrofuran, Dioxan, Dimethoxyethan, Dichlormethan oder Acetonitril und mindestens 3 Äquivalenten Pyridin vorlegt, bei Temperaturen zwischen -20° C und Raumtemperatur 1 Äquivalent Phosphoroxychlorid zutropft und gegebenenfalls bei erhöhter Temperatur, wie beispielsweise 60° C, nachrührt.

Außerdem bevorzugt werden die den Carbonsäuren der Formeln (I-10), (I-13) und (I-19) entsprechenden O-Acyl-isoharnstoffe eingesetzt. Sie können erhalten werden, indem man 1 Äquivalent der entsprechenden Carbonsäuren der Formeln (I-10), (I-13) oder (I-19) und 1 Äquivalent des entsprechenden Amins der Formel (VII) und gegebenenfalls 1 Äquivalent 1-Hydroxybenzotriazol als Katalysator in einem Lösungsmittel, wie beispielsweise Dichlormethan, Dimethylformamid, Dioxan, Tetrahydrofuran und Acetonitril, löst und bei Temperaturen zwischen -20° C und 0° C eine Lösung von 1 Äquivalent Dicyclohexylcarbodiimid in einem Lösungsmittel zutropft und gegebenenfalls bei Temperaturen zwischen 0° und 30° C nachrührt.

Ebenfalls bevorzugt werden die den Carbonsäuren der Formeln (I-10), (I-13) und (I-19) entsprechenden aktivierten Ester. Sie können erhalten werden, indem man 1 Äquivalent der entsprechenden Carbonsäuren der Formeln (I-10), (I-13) und (I-19) mit 1 Äquivalent eines Phenols, wie beispielsweise p-Nitrophenol oder Trichlorphenol bzw. mit 1 Äquivalent einer N-Hydroxyverbindung, wie z. B. N-Hydroxysuccinimid, 1 Äquivalent Dicyclohexylcarbodiimid in einem Lösungsmittel, wie beispielsweise Dichlormethan, Essigester, Dimethylformamid, Tetrahydrofuran, Dioxan, Dimethoxyethan oder Acetonitril, bei Temperaturen zwischen -20° C und +20° C umsetzt. Der so erhaltene Ester wird isoliert und mit 1 Äquivalent des entsprechenden Amins in einem Lösungsmittel, wie beispielsweise Dimethylformamid, Dimethylsulfoxid, Wasser und Acetonitril, bei Raumtemperatur umgesetzt.

Ebenfalls bevorzugt werden die aus den Carbonsäuren der Formeln (I-10), (I-13) und (I-19) mit Imidchloriden, wie z. B. dem Dimethylformimidium, aktivierten Derivate eingesetzt. Sie können erhalten werden, indem man beispielsweise 1 Äquivalent Dimethylformamid in einem Lösungsmittel, wie beispielsweise Acetonitril oder Dichlormethan, bei -20° C mit 1 Äquivalent Oxalylchlorid versetzt, etwa 15 Minuten bei -20° C nachrührt, eine Lösung von 1 Äquivalent der entsprechenden Carbonsäure der Formeln (I-10), (I-13) oder (I-19) in einem Lösungsmittel, wie beispielsweise Acetonitril bei -20° C zugibt und etwa 15 Minuten bei -20° C nachrührt. Anschließend wird eine Lösung von 1 Äquivalent des entsprechenden Amins der Formel (VII) und 1 Äquivalent einer tertiären Base, wie beispielsweise Pyridin oder Triethylamin, in einem Lösungsmittel, wie beispielsweise Acetonitril oder Pyridin, bei -20° C zugegeben, auf Raumtemperatur erwärmt und bei Raumtemperatur nachgerührt.

Als Lösungsmittel kommen für das erfindungsgemäße Verfahren (a) alle üblichen inerten organischen Lösungsmittel in Frage. Hierzu gehören vorzugsweise Ether, wie Dioxan, Tetrahydrofuran und Dimethoxyethan; Nitrile, wie Acetonitril; Amide, wie Dimethylformamid oder Sulfoxide, wie Dimethylsulfoxid.

Das erfindungsgemäße Verfahren (a) wird in Gegenwart einer Base durchgeführt. Hierzu können alle üblicherweise verwendbaren anorganischen und organischen Basen eingesetzt werden, wie insbesondere Alkalimetallcarbonate, wie Natriumcarbonat, Kaliumcarbonat; Alkalimetallhydroxide, wie Natriumhydroxid; Alkalimetallalkoholate, wie Kalium-tert.-butylat; Alkalimetallhydride, wie Natriumhydrid; tertiäre Amine, wie Triethylamin, Diazabicyclooctan (DABCO) oder Diazabicycloundecen (DBU).

Das erfindungsgemäße Verfahren (a) wird gegebenenfalls in Gegenwart eines Phasentransferkatalysators durchgeführt. Beispielsweise genannt seien quaternäre Ammoniumsalze, wie Tetrabutylammoniumbromid oder Methyl-trioctylammoniumchlorid.

Die Reaktionstemperaturen können bei der Durchführung des erfindungsgemäßen Verfahrens (a) in einem größeren Bereich variiert werden. Im allgemeinen arbeitet man bei Temperaturen zwischen -80° C und 100° C, vorzugsweise bei Temperaturen zwischen -20° C und 60° C.

Bei der Durchführung des erfindungsgemäßen Verfahrens (a) setzt man pro Mol 1-Amino-cyclopropancarbonsäurederivat der Formel (II) im allgemeinen 1 bis 3 Mol, vorzugsweise 1 bis 2 Mol an Alkylierungsmittel der Formel (III) ein.

Als Verdünnungsmittel kommen für das erfindungsgemäße Verfahren (b) Wasser und inerte organische Lösungsmittel in Frage. Hierzu gehören vorzugsweise Alkohole, wie Methanol und Ethanol; Nitrile, wie Acetonitril; Ester, wie Ethylacetat; Ether, wie Dioxan; Amide, wie Dimethylformamid; Chlorkohlenwasserstoffe, wie Methylenchlorid und Chloroform, oder tertiäre Amine, wie Pyridin.

Das erfindungsgemäße Verfahren (b) wird gegebenenfalls in Gegenwart eines sauren oder basischen Katalysators durchgeführt. Beispielsweise genannt seien Schwefelsäure, Essigsäure , p-Toluolsulfonsäure und Bortrifluorid.

Die Reaktionstemperaturen können bei der Durchführung des erfindungsgemäßen Verfahrens (b) in einem größeren Bereich variiert werden. Im allgemeinen arbeitet man bei Temperaturen zwischen 0° C und 170° C, vorzugsweise bei Raumtemperatur bis Rückflußtemperatur.

Bei der Durchführung des Verfahrens (b) setzt man pro Mol 1-Aminocyclopropancarbonsäurederivat der Formel (IV) im allgemeinen 1 bis 2 Mol, vorzugsweise 1 Mol, an Olefinen der Formel (V) ein.

Die erfindungsgemäße Hydrierung gemäß Verfahren c), h) und m) erfolgt in üblicher Weise in Gegenwart von Wasserstoff und eines üblicherweise verwendbaren Katalysators, wie beispielsweise Palladium/Kohlenstoff.

Als Lösungsmittel kommen für die erfindungsgemäße Hydrierung polare organische Lösungsmittel in Frage. Hierzu gehören vorzugsweise Alkohole, wie Methanol, Ethanol und Isopropanol.

Die Reakton wird im allgemeinen bei 0° C bis 80° C, vorzugsweise bei 20° C bis 40° C durchgeführt.

Die Hydrierung gemäß Verfahren c), h) und m) kann bei Normaldruck aber auch bei erhöhtem Druck durchgeführt werden. Im allgemeinen arbeitet man bei Drucken zwischen 1 und 50 bar, vorzugsweise zwischen 1 und 20 bar.

Die erfindungsgemäße Hydrolyse gemäß Verfahren d), g) und l) erfolgt in üblicher Weise in Gegenwart einer wäßrigen Base und gegebenenfalls in Gegenwart eines wäßrigen oder mit Wasser mischbaren organischen Lösungsmittels. Vorzugsweise verwendet man Wasser, Alkohole, wie Methanol und Ethanol, oder Mischungen von Wasser und Alkoholen oder Ethern, wie Dioxan.

Als Base kommen für die erfindungsgemäße Hydrolyse gemäß Verfahren d), g) und l) alle üblichen Hydroxid-enthaltenden Basen in Frage. Hierzu gehören vorzugsweise Alkalimetallhydroxide, wie Natriumhydroxid und Kaliumhydroxid, sowie Erdalkalimetallhydroxide, wie Bariumhydroxid.

Die Temperaturen können bei der Durchführung der erfindungsgemäßen Hydrolyse gemäß Verfahren d), g) und l) in einem größeren Bereich variiert werden. Im allgemeinen arbeitet man bei Temperaturen zwischen 0° C und 120° C, vorzugsweise bei Temperaturen zwischen 20° C und 80° C.

Als Lösungsmittel kommen für die erfindungsgemäßen Aminolyse gemäß den Verfahren e), i) und n) alle üblichen mit Wasser mischbaren organischen Lösungsmittel in Frage. Vorzugsweise verwendet man Alkohole, wie Methanol, Mischungen von Alkoholen, wie Methanol und Wasser sowie Glycol.

Die Reaktionstemperaturen können bei der Durchführung der erfindungsgemäßen Aminolyse gemäß den Verfahren e), i) und n) in einem größeren Bereich variiert werden. Im allgemeinen arbeitet man bei Temperaturen zwischen -30° C und 150° C, vorzugsweise bei zwischen 0° C und Rückflußtemperatur.

Bei der Durchführung der erfindungsgemäßen Verfahren e), i) und l) arbeitet man im allgemeinen mit einem großen Überschuß des Amins der Formel (VI), vorzugsweise mit einem dreifachen Überschuß.

Als Verdünnungsmittel kommen für die erfindungsgemäßen Verfahren f), k) und o) Wasser und organische Lösungsmittel in Frage. Hierzu gehören vorzugsweise Chlorkohlenwasserstoffe, wie Dichlormethan; Ester, wie Essigester; Ether, wie Dioxan, Tetrahydrofuran und Dimethoxyethan; Nitrile, wie Acetonitril; Amide, wie Dimethylformamid.

Die erfindungsgemäßen Verfahren f), k), und o) werden gegebenenfalls in Gegenwart einer Base durchgeführt. Hierzu gehören alle üblicherweise verwendbaren anorganischen und organischen Basen, wie insbesondere Kaliumcarbonat, Triethylamin, N-Methylmorpholin und Pyridin.

Die erfindungsgemäßen Verfahren f), k) und o) werden gegebenenfalls in Gegenwart eines Katalysators durchgeführt. Beispielsweise genannt seien Dimethylformamid, 4-Dimethylaminopyridin und 1-Hydroxybenzotriazol.

Die Reaktionstemperaturen können bei der Durchführung der erfindungsgemäßen Verfahren f), k) und o) in einem größeren Bereich variiert werden. Im allgemeinen arbeitet man bei Temperaturen zwischen -60° C und 40° C, vorzugsweise bei Temperaturen zwischen -20° C und 20° C.

Bei der Durchführung der erfindungsgemäßen Verfahren f), k) und o) setzt man pro Mol der carboxyaktivierten Derivate der Carbonsäuren der Formeln (I-10), (I-13) bzw. (I-19) im allgemeinen 1 bis 2 Mol, vorzugsweise 1 Mol Amin der Formel (VII) und im allgemeinen 1 bis 2 Mol Base ein.

Als Lösungsmittel kommen für das erfindungsgemäße Verfahren p) alle üblichen mit Wasser mischbaren organischen Lösungsmitel in Frage. Hierzu gehören vorzugsweise Ether, wie Dioxan, Tetrahydrofuran und Dimethoxyethan sowie Nitrile, wie Acetonitril.

Das erfindungsgemäße Verfahren p) wird durch Behandlung mit einer Säure durchgeführt. Beispielsweise genannt seien Salzsäure oder wasserfreier Chlorwasserstoff.

Die Reaktionstemperaturen können bei der Durchführung des erfindungsgemäßen Verfahrens p) in einem größeren Bereich variiert werden. Im allgemeinen arbeitet man bei Temperaturen zwischen -20° C und 100° C, vorzugsweise bei Temperaturen zwischen 0° C und 20° C.

Bei der Durchführung des erfindungsgemäßen Verfahrens p) arbeitet man im allgemeinen mit einem großen Überschuß an Salzsäure, vorzugsweise mit einem drei-bis fünffachen Überschuß.

Die erfindungsgemäße Totalhydrolyse gemäß Verfahren q) wird mit einer starken wäßrigen Säure durchgeführt. Hierzu gehören vorzugsweise die Salzsäure und Schwefelsäure.

Die Reaktionstemperaturen können bei der Durchführung der Totalhydrolyse gemäß Verfahren q) in einem größeren Bereich variiert werden. Im allgemeinen arbeitet man bei Temperaturen zwischen 80° C und 150° C, vorzugsweise bei der entsprechenden Rückflußtemperatur.

Als Verdünnungsmitel kommen für das erfindungsgemäße Verfahren r) Wasser und alle üblichen mit Wasser mischbaren organischen Lösungsmittel in Frage Vorzugsweise verwendet man Alkohole, wie Methanol und Ethanol, und Ether, wie Dioxan und Tetrahydrofuran.

Das erfindungsgemäße Verfahren r) wird in Gegenwart einer Base durchgeführt. Hierzu können alle üblicherweise verwendbaren Basen eingesetzt werden, wie insbesondere Alkali-und Erdalkalimetallhydroxide, wie Kaliumhydroxid oder Bariumhydroxid, Alkalimetallcarbonate, wie Kaliumcarbonat oder Natriumcarbonat, sowie Ammoniak und Natriumboronat.

Die Reaktionstemperaturen können bei der Durchführung des erfindungsgemäßen Verfahrens r) in einem größeren Bereich variiert werden. Im allgemeinen arbeitet man bei Temperaturen zwischen 0° C und 100° C, vorzugsweise bei Temperaturen zwischen 0° C und 60° C.

Als Lösungsmittel kommen für das erfindungsgemäße Verfahren s) Wasser und alle üblichen inerten organischen Lösungsmittel in Frage. Hierzu gehören vorzugsweise Kohlenwasserstoffe, wie Toluol; Chlorkohlenwasserstoffe, wie Methylenchlorid und Chloroform; Ether, wie Tetrahydrofuran und Dioxan; Nitrile, wie Acetonitril; Amide, wie Dimethylformamid; sowie Ester, wie Ethylacetat.

Das erfindungsgemäße Verfahren s) wird in Gegenwart einer Base durchgeführt. Hierzu können alle üblicherweise verwendbaren anorganischen und organischen Basen eingesetzt werden, wie insbesondere Alkalimetallhydroxide, wie Natriumhydroxid; Alkalimetallcarbenate, wie Natrium-und Kaliumcarbonat; Erdalkalioxide, wie Magnesiumoxide; teriäre Amine, wie Triethylamin und Pyridin.

Das erfindungsgemäße Verfahren s) wird gegebenenfalls in Gegenwart eines Katalysators durchgeführt. Beispielsweise genannt seien Dimethylformamid und Dimethylaminopyridin.

Die Reaktionstemperaturen können bei der Durchführung des erfindungsgemäßen Verfahrens s) in einem größeren Bereich variiert werden. Im allgemeinen arbeitet man bei Temperaturen zwischen -20° C und 120° C, vorzugsweise bei Temperaturen zwischen -20° C und 40° C.

Bei der Durchführung des erfindungsgemäßen Verfahrens s) setzt man pro Mol der Verbindung der Formel (I-27). im allgemeinen 1 bis 2 Mol, vorzugsweise 1 Mol an carboxy-aktivierten Derivaten der Carbonsäuren der Formel (VIII) ein.

Zur Herstellung von Säureadditions-Salzen der Verbindungen der Formel (I) kommen alle diejenigen Säuren in Frage, die zu physiologisch verträglichen Salzen führen. Vorzugsweise verwendbar sind diejenigen Säuren, die bereits im Zusammenhang mit der Beschreibung der erfindungsgemäßen Stoffe als bevorzugt zu addierenden Säuren genannt wurden.

Die Säureadditions-Salze der Verbindungen der Formel (I) lassen sich in einfacher Weise nach üblichen Salzbildungsmethoden herstellen. Im allgemeinen verfährt man so, daß man eine Verbindung der Formel (I) in einem geeigneten Verdünnungsmittel löst und dann eine Säure hinzufügt. die Isolierung erfolgt in bekannter Weise, zum Beispiel dadurch, daß man das Salz abfiltriert und gegebenenfalls durch Waschen mit einem organischen Lösungsmittel reinigt.

Zur Herstellung von Alkali-, Erdalkali-und gegebenenfalls substituierten Ammoniumsalzen der Verbindungen der Formel (I) kommen alle diejenigen Alkali-und Erdalkalihydroxide, -carbonate oder -hydrogencarbonate oder Amine sowie Ammoniumhydroxid in Frage, die zu physiologisch verträglichen Salzen führen. Vorzugsweise verwendbar sind diejenigen Basen, die bereits im Zusammenhang mit der Beschreibung der erfindungsgemäßen Stoffe als bevorzugt genannt wurden.

Die Alkali-, Erdalkali-und gegebenenfalls substituierten Ammoniumsalze der Verbindungen der Formel - (I) lassen sich in einfacher Weise nach üblichen Salzbildungsmethoden herstellen. Im allgemeinen verfährt man so, daß man eine Verbindung der Formel (I) in einem geeigneten Verdünnungsmittel löst und dann die entsprechenden Base hinzufügt. Die Isolierung erfolgt in bekannter Weise, zum Beispiel dadurch, daß man das Salz abfiltriert und gegebenenfalls durch Waschen mit einem organischen Lösungsmittel reinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel, vor allem als Fungizide, geeignet.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt.

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilage nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria bassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Als Schädlingsbekämpfungsmittel können die erfindungsgemäßen Wirkstoffe mit besonders gutem Erfolg zur Bekämpfung von Plasmopara-Arten, wie beispielsweise Plasmopara viticola an Reben eingesetzt werden. Außerdem zeigen die erfindungsgemäßen Wirkstoffe in entsprechenden Aufwandmengen bzw. Konzentrationen auch eine fungizide Wirkung gegen Pyricularia-Arten, wie Pyricularia oryzae am Reis sowie eine pflanzenwachstumsregulierende Wirkung und eine systemische Wirkung insbesondere gegen Getreidemehltau. Ebenso könnnen die erfindungsgemäßen Wirkstoffe als Synergisten für Insektizide eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt-und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z. B.

gebrochene und fraktionierte natürliche Gesteine wie Calcit, Marmor, Bims, Spiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und bodenstrukturverbesserungsmitteln.

Wie Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1:

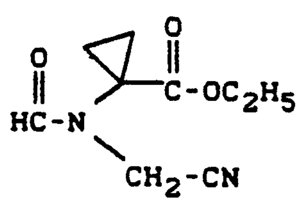

(Verfahren a)

In eine Lösung von 90,0 g (0,573 mol) 1-[N-Formyl-amino]-cyclopropancarbonsäureethylester in 500 ml absolutem Tetrahydrofuran gibt man 18 g (0,056 mol) Tetrabutylammoniumbromid und bei 20° C (Kühlung erforderlich) portionsweise 75 g (0,668 mol) Kalium-tert.-butylat. In die gelbe Suspension werden dann bei 20° C 99g (1,31 mol) Chloracetonitril getropft und die Mischung 24 Stünden bei Raumtemperatur gerührt. Das Reaktionsgemisch wird dreimal mit je 100 ml Wasser gewaschen, die organische Phase getrocknet und im Vakuum eingedampft. Der Rückstand (130 g) wird an 750 g Kieselgel mit Chloroform/Methanol (100:1)

chromatographiert. Es resultiert ein gelbes Öl (57 g). Nach Behandlung mit Ether erhält man 49,2 g (43,8 % der Theorie) 1-[N-Cyanmethyl-N-formyl-amino]-cyclopropancarbonsäureethylester vom Schmelzpunkt 65-68° C.

Beispiel 2:

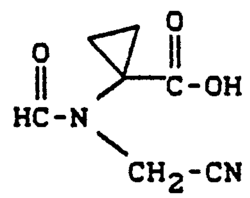

(Verfahren d)

In eine Lösung von 19,6 g (0,1 mol) 1-[N-Cyanmethyl-N-formyl-amino]-cyclopropancarbonsäureethylester in 50 ml Methanol tropft man eine Lösung von 6,1 g (0,11 mol) Kaliumhydroxid in 50 ml Wasser und rührt 7 Stunden bei 70° C. Das Methanol wird im Vakuum abdestilliert und die verbleibende wässrige Lösung über eine Säule von Lewatit S 100, $H^+$-Form filtriert. Das Eluat wird eingedampft, der Rückstand in Methylenchlorid aufgeschlämmt, filtriert und getrocknet. Man erhält 14,3 g (85 % der Theorie) 1-[N-Cyanmethyl-N-formyl-amino]-cyclopropancarbonsäure vom Schmelpunkt 185-191° C.

Beispiel 3:

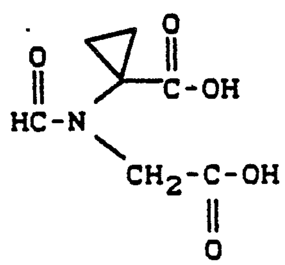

(Verfahren 1)

Eine Lösung von 24,3 g (0,1 mol) 1-[N-Ethoxycarbonylmethyl-N-formyl-amino]-cyclopropancarbonsäureethylester und 12,3 g (0,22 mol) Kaliumhydroxid in 75 ml Methanol und 75 ml Wasser wird 7 Stunden bei 70° C gerührt. Nach Abkühlung wird die Reaktionslösung dreimal mit je 50 ml Chloroform gewaschen, die wässrige Phase eingeengt, auf eine Säule von Lewatit S 100, $H^+$-Form, aufgetragen und mit Wasser eluiert. Das Eluat wird eingedampft und der Rückstand aus Aceton kristallisiert. Man erhält 13,3 g - (71 % der Theorie) 1-[N-Carboxymethyl-N-formyl-amino]-cyclopropancarbonsäure vom Schmelzpunkt 160° C (Zersetzung).

Beispiel 4:

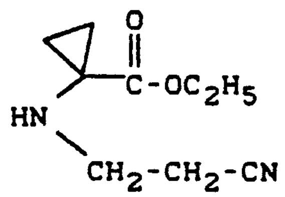

(Verfahren p)

Eine Lösung von 147,1 g (0,7 mol) 1-[N-(2-Cyanethyl)-N-formyl-amino]-cyclopropancarbonsäureethylester in 1 l Tetrahydrofuran wird mit 289 ml (3,5 mol) konzentrierter Salzsäure versetzt und 5 Tage bei Raumtemperatur gerührt. Man dampft das Reaktionsgemisch im Vakuum ein, versetzt den Rückstand mit 500 ml Toluol und 141,7 g (1,4 mol) Triethylamin und rührt 1 Stunde bei Raumtemperatur. Man filtriert und dampft das Filtrat im Vakuum ein. Der Rückstand wird an Kieselgel mit Essigester chromatographiert. Dabei resultiert ein Öl, das zweimal im Vakuum destilliert wird. Man erhält 36,4 g (28,5 % der Theorie) 1-[N-(2-Cyanethyl)-amino]-cyclopropancarbonsäureethylester vom Siedepunkt 90-100° /0,05 mbar.

Beispiel 5:

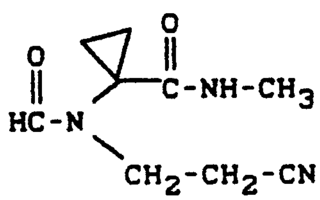

(Verfahren e)

In eine Lösung von 11,5 g (0,05 mol) 91 prozentigem 1-[N-(2-Cyanethyl)-N-formyl-amino]-cyclopropancarbonsäureethylester in 100 ml absolutem Methanol leitet man bei -10° C 2 Stunden lang Methylamin ein. Nach Erwärmung auf Raumtemperatur rührt man 20 Stunden nach. Die Lösung wird im Vakuum eingedampft, der Rückstand mit Petrolether aufgeschlämmt, abgesaugt und getrocknet. Man erhält 9,3 g (95,3 % der Theorie) 1-[N-(2-Cyanethyl)-N-formyl-amino]-cyclopropancarbonsäuremethylamid vom Schmelzpunkt 105 -108° C.

Beispiel 6:

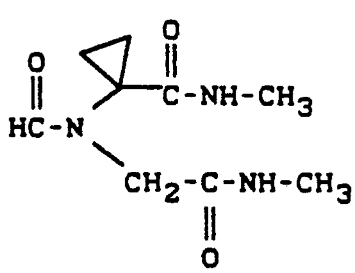

(Verfahren n)

In eine Lösung von 14,6 g (0,06 mol) 1-[N-Ethoxycarbonylmethyl-N-formyl-amino]-cyclopropancarbonsäureethylester in 100 ml trockenem Methanol leitet man bei -10° C 2 Stunden lang Methylamin ein. Nach Erwärmen auf Raumtemperatur rührt man 20 Stunden nach. Die Lösung wird im Vakuum eingedampft, der Rückstand mit Essigester versetzt, abgesaugt und getrocknet. Man erhält 7,5 g (58,6 % der Theorie) 1-[N-Formyl-N-methylcarbamoylmethyl-amino]-cyclopropancarbonsäuremethylamid von Schmelzpunkt 129-135° C.

Beispiel 7:

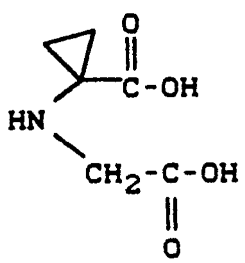

(Verfahren q)

14,6 g (0,06 mol) 1-[N-Ethoxycarbonylmethyl-N-formyl-amino]-cyclopropancarbonsäureethylester werden mit 75 ml konzentrierter Salzsäure 5 Stunden unter Rückfluß erhitzt. Die Reaktionslösung wird im Vakuum eingedampft, der Rückstand in 50 ml Methanol gelöst, tropfenweise bei Raumtemperatur mit 7,0 g (0,12 mol) Propylenoxid versetzt und 2 Stunden bei Raumtemperatur gerührt. Man saugt den Niederschlag ab, wäscht mit Ether und trocknet. Man erhält 7,3 g (76,5 % der Theorie) 1-[N-Carboxymethyl-amino]-cyclopropancarbonsäure als farblose Kristalle vom Zersetzungspunkt 230° C.

Beispiel 8:

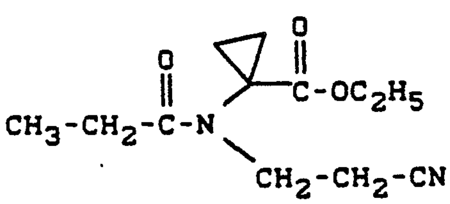

(Verfahren s)

3,6 g (0,02 mol) 1-[N-(2-Cyanethyl)-amino]-cyclopropancarbonsäureethylester, 40 g (0,04 mol) Triethylamin und 0,2 g (0,002 mol) 4-Dimethylaminopyridin werden in 50 ml Methylenchlorid gelöst, bei 0° C tropfenweise mit einer Lösung von 3,7 g (0,04 mol) Propionylchlorid in 10 ml Methylenchlorid versetzt und die Mischung 20 Stunden bei Raumtemperatur gerührt. Nach Verdünnen mit 100 ml Methylenchlorid wird dreimal mit je 50 ml Wasser gewaschen, die organische Phase getrocknet und eingedampft und der Rückstand im Vakuum destilliert (Kugelrohr). Man erhält 3,5 g (73,4 % der Theorie) 1-[N-(2-Cyanehtyl)-N-propionyl-amino]-cyclopropancarbonsäureethylester als gelbes Öl mit einem Siedepunkt von 160° C/0,2 mbar.

Beispiel 9:

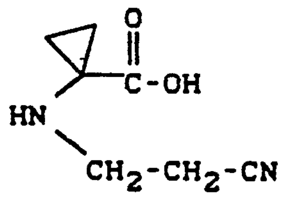

(Verfahren b)

Eine Lösung von 2,7 g (0,02 Mol) 1-Amino-cyclopropancarbonsäure-hydrochlorid in 20 ml Wasser und 20 ml 2 m Natronlauge wird bei 20° C mit 1,1 g (0,022 Mol) Acrylnitril versetzt und 2 Stunden geschüttelt. Nach Zugabe von 1,2 g (0,02 Mol) Eisessig wird das Reaktionsgemisch bis auf 2 ml im Vakuum eingeengt und mit 10 ml Ethanol versetzt. Nach 1 Stunde bei 0° wird der Niederschlag abgesaugt, mit wenig Wasser gewaschen und getrocknet. Man erhält 2,4 g (78 % der Theorie) 1-[N-(2-Cyanethyl)-amino]-cyclopropancarbonsäure mit dem Zersetzungspunkt 190° C.

In analoger Weise und entsprechend den erfindungsgemäßen Verfahren werden die nachfolgenden Verbindungen der Formel (I)

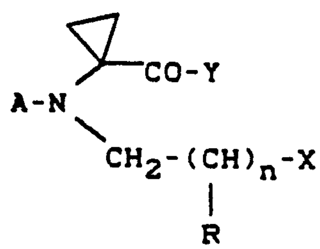

(I)

erhalten:

| Bsp. Nr. | A | Y | R | n | X | Physikalische Daten |
|---|---|---|---|---|---|---|
| 10 | HCO- | $-OC_2H_5$ | - | 0 | $-CH=CH_2$ | $n_D^{20}$ :1,4702 |
| 11 | " | " | - | 0 | $-COOC_2H_5$ | $n_D^{20}$ :1,4605 |
| 12 | " | -OH | - | 0 | $-CH=CH_2$ | Fp: 122-125° C |
| 13 | " | $-NH-CH_3$ | - | 0 | " | Fp: 112-214° C |
| 14 | " | $-OC_2H_5$ | - | 0 | $-CH=CH-CH_3$ | $Kp_{0,2}$:95-100° C |
| 15 | " | -OH | - | 0 | " | Fp: 106-109° C |
| 16 | " | $-NH-CH_3$ | - | 0 | " | Fp: 105-108° C |
| 17 | " | $-OC_2H_5$ | H | 1 | -CN | $n_D^{20}$ :1.4720 |
| 18 | " | " | H | 2 | " | $Kp_{0,3}$:130-140° |
| 19 | " | -OH | H | 1 | " | Fp: 155-159° C |
| 20 | " | " | H | 2 | " | Fp: 123-125° C |
| 21 | " | $-NH-CH_3$ | H | 2 | " | Fp: 95-99° C |
| 22 | " | $-OC_8H_{17}$ | H | 1 | " | $n_D^{20}$ :1,4675 |
| 23 | 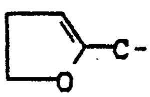 C-O- | $-OC_2H_5$ | H | 1 | " | $Kp_{0,2}$:160° C |
| 24 | 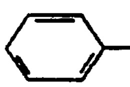 CO- | " | | | " | $Kp_{0,3}$: 180° C |
| 25 | H | -OH | H | 1 | -COOH | Fp:198-200° C |
| 26 | $CH_3-CO-$ | -OH | H | 1 | -CN | Fp: 144-146 |
| 27 | $ClCH_2-CO-$ | -OH | H | 1 | -CN | Öl |

Verwendungsbeispiele

Im nachfolgenden Verwendungsbeispiel werden die nachfolgend angegebenen Substanzen als Vergleichsverbindungen eingesetzt:

(A) 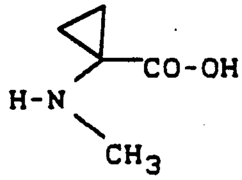

(B) 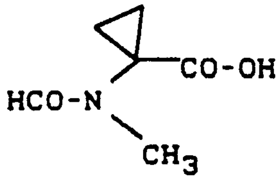

(C) 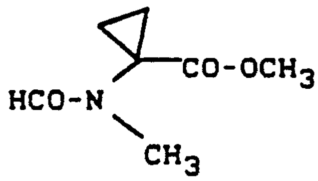

Beispiel A

Plasmopara-Test (Reben) / protektiv

Lösungsmittel: 4,7 Gewichtsteile Aceton

Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22° C und 100 % relativer Luftfeuchtigkeit. Anschließend werden die Pflanzen 5 Tage im Gewächshaus bei 22° C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Tecknik zeigen in diesem Test

Verbindungen gemäß den Herstellungsbeispielen.

**Ansprüche**

1. Substituierte 1-Amino-cyclopropancarbonsäurederivate der Formel (I)

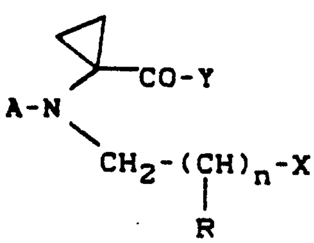 (I)

in welcher

Y für die Gruppierung $OR^1$ oder $-NR^2R^3$ steht, wobei

$R^1$ für Wasserstoff, Alkyl oder für gegebenenfalls substituiertes Benzyl steht,

$R^2$ für Wasserstoff oder Alkyl steht,
$R^3$ für Wasserstoff oder Alkyl steht, oder
$R^2$ und $R^3$ gemeinsam mit dem Stickstoffatom, an das sie gebunden sind, für einen gegebenenfalls substituierten mehrgliedrigen Ring stehen, der weitere gleiche oder verschiedene Heteroatome enthalten kann,
A für Wasserstoff oder einen Acylrest steht,
R für Wasserstoff oder Alkyl steht,
n für die Zahlen 0,1 oder 2 steht und
X für Alkenyl, Alkinyl, für Hydroxycarbonyl, Alkoxycarbonyl, Aminocarbonyl, Alkylaminocarbonyl, Dialkylaminocarbonyl oder Cyano steht,
und deren pflanzenverträgliche Säureadditionssalze und deren physiologisch verträgliche gegebenenfalls substituierten Ammonium-, Alkali-und Erdalkali-Salze.

2. Substituierte 1-Amino-cyclopropancarbonsäurederivate der Formel (I) gemäß Anspruch 1, in welcher
Y für die Gruppierung $OR^1$ oder $-NR^2R^3$ steht, in welcher
$R^1$ für Wasserstoff, für geradkettiges oder verzweigtes Alkyl mit 1 bis 10 Kohlenstoffatomen, für gegebenenfalls gleich oder verschieden, ein-bis fünffach durch Halogen, Alkyl mit 1 bis 4 Kohlenstoffatomen und Halogenalkyl mit 1 oder 2 Kohlenstoffatomen und 1 bis 5 gleichen oder verschiedenen Halogenatomen substituiertes Benzyl steht,
$R^2$ für Wasserstoff oder für geradkettiges oder verzweigtes Alkyl mit 1 bis 6 Kohlenstoffatomen steht und
$R^3$ für Wasserstoff oder für geradkettiges oder verzweigtes Alkyl mit 1 bis 6 Kohlenstoffatomen steht, oder
$R^2$ und $R^3$ gemeinsam mit dem Stickstoffatom, an das sie gebunden sind, für einen gegebenenfalls substituierten 5-bis 7-gliedrigen Ring stehen, der weitere gleiche oder verschiedene Heteroatome enthalten kann,
A für Wasserstoff oder für den Rest W-CO-steht, wobei
W für Wasserstoff, für geradkettiges oder verzweigtes Alkyl mit 1 bis 6 Kohlenstoffatomen, für Alkenyl oder Alkinyl mit 2 bis 4 Kohlenstoffatomen, für Cycloalkyl mit 3 bis 6 Kohlenstoffatomen, für Halogenalkyl mit 1 oder 2 Kohlenstoffatomen und 1 bis 5 gleichen oder verschiedenen Halogenatomen, für Alkoxyalkyl oder Alkoxycarbonyl mit jeweils 1 bis 4 Kohlenstoffatomen in jedem Alkylteil bzw. Alkoxyteil, für den Rest $CH_3SO_m$-$CH_2$-steht, in welchem
m für die Zahlen 0, 1 oder 2 steht,
weiterhin für Phenyl, Benzyl oder für einen 5-oder 6-gliedrigen heterocyclischen Ring mit 1 oder 2 weiteren, gleichen oder verschiedenen Heteroatomen, steht,
R für Wasserstoff oder geradkettiges oder verzweigtes Alkyl mit 1 bis 4 Kohlenstoffatomen steht,
n für die Zahlen 0, 1 oder 2 steht, und
X für Alkenyl oder Alkinyl mit 2 bis 4 Kohlenstoffatomen, für Hydroxycarbonyl, für Alkoxycarbonyl mit 1 bis 4 Kohlenstoffatomen im Alkoxyteil, für Aminocarbonyl, für Alkylaminocarbonyl oder Dialkylaminocarbonyl mit jeweils 1 bis 4 Kohlenstoffatomen in jedem Alkylteil oder für Cyano steht.

3. Substituierte 1-Amino-cyclocarbonsäure-derivate der Formel (I) gemäß Anspruch 1, in welcher
Y für die Gruppierung $OR^1$ oder $NR^2R^3$ steht, wobei
$R^1$ für Wasserstoff, für geradkettiges oder verzweigtes Alkyl mit 1 bis 8 Kohlenstoffatomen oder für gegebenenfalls ein-bis dreifach, gleich oder verschieden durch Fluor, Chlor, Brom, Methyl, Ethyl, n-und i-Propyl und Trifluormethyl substituiertes Benzyl steht,
$R^2$ für Wasserstoff, Methyl, Ethyl, n-und i-Propyl oder n-, i-, s-und t-Butyl steht, und
$R^3$ für Wasserstoff, Methyl, Ethyl, n-und i-Propyl oder n-, i-, s-und t-Butyl steht, oder
$R^2$ und $R^3$ gemeinsam mit dem Stickstoffatom, an das sie gebunden sind, für Pyrrolidin, Piperidin, Morpholin oder N-Methylpiperazin stehen,
A für Wasserstoff oder für den Rest W-CO-steht, in welchem
W für Wasserstoff, Methyl, Ethyl, n-und i-Propyl, n-, i-, s-und t-Butyl, Vinyl, Ethinyl, Cyclopropyl, Cyclopentyl, Cyclohexyl, Trifluormethyl, Chlormethyl, Dichlormethyl, Trichlormethyl, Methoxymethyl, 2-Methoxyethyl, 2-Ethoxyethyl, Methoxycarbonyl, Ethoxycarbonyl, Methylthiomethyl, Methylsulfinylmethyl, Methylsulfonylmethyl, Phenyl, Benzyl, Furan-2-yl oder Isoxazol-5-yl steht,
R für Wasserstoff, Methyl, Ethyl oder n-und i-Propyl steht,
n für die Zahlen 0, 1 oder 2 steht, und
X für Vinyl, Ethinyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht.

4. Substituierte 1-Amino-cyclopropancarbonsäurederivate der Formel (I) gemäß den Ansprüchen 1 bis 3, in welcher
Y für die Gruppierung $OR^1$ oder $NR^2R^3$ steht, in welcher

$R^1$ für Wasserstoff, für geradkettiges oder verzweigtes Alkyl mit 1 bis 8 Kohlenstoffatomen oder für gegebenenfalls ein-bis dreifach, gleich oder verschieden durch Fluor, Chlor, Brom, Methyl, Ethyl, n-Propyl, iso-Propyl und Trifluormethyl substituiertes Benzyl steht,
$R^2$ für Wasserstoff, Methyl, Ethyl, n-Propyl, iso-Propyl, n-, i-, s-oder t-Butyl steht,
$R^3$ für Wasserstoff, Methyl, Ethyl, n-Propyl, iso-Propyl, n-, i-, s-oder t-Butyl steht oder
$R^2$ und $R^3$ gemeinsam mit dem Stickstoffatom, an das sie gebunden sind, für Pyrrolidin, Piperidin, Morpholin oder N-Methylpiperazin stehen,
A für Wasserstoff oder den Rest HCO-steht,
R für Wasserstoff, Methyl, Ethyl oder n-und i-Propyl steht,
n für die Zahlen 0, 1 oder 2 steht, und
X für Vinyl, Ethinyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder für Cyano steht.

5. Verfahren zur Herstellung von substituierten 1-Amino-cyclopropancarbonsäurederivaten der Formel - (I)

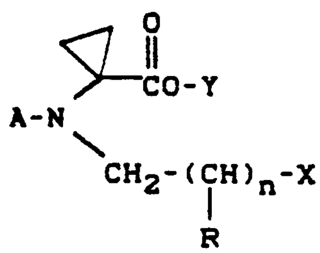

(I)

in welcher
Y für die Gruppierung $OR^1$ oder $-NR^2R^3$ steht, wobei
$R^1$ für Wasserstoff, Alkyl oder für gegebenenfalls substituiertes Benzyl steht,
$R^2$ für Wasserstoff oder Alkyl steht und
$R^3$ für Wasserstoff oder Alkyl steht, oder
$R^2$ und $R^3$ gemeinsam mit dem Stickstoffatom, an das sie gebunden sind, für einen gegebenenfalls substituierten mehrgliedrigen Ring stehen, der weitere gleiche oder verschiedene Heteroatome enthalten kann,
A für Wasserstoff oder einen Acylrest steht,
R für Wasserstoff oder Alkyl steht,
n für die Zahlen 0,1 oder 2 steht und
X für Alkenyl, Alkinyl, für Hydroxycarbonyl, Alkoxycarbonyl, Aminocarbonyl, Alkylaminocarbonyl, Dialkylaminocarbonyl oder Cyano steht,
und deren pflanzenverträglichen Säureadditionssalze und gegebenenfalls substituierten Ammonium-, Alkali- und Erdalkali-Salze, dadurch gekennzeichnet, daß man

a) die erfindungsgemäßen substituierten 1-Amino-cyclopropancarbonsäurederivate der Formel (I-1)

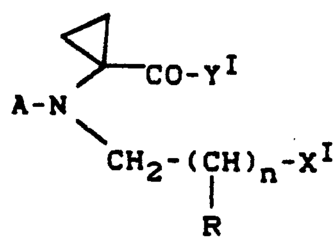

(I-1)

in welcher
R und n die oben angegebenen Bedeutungen haben,
$A^I$ für einen Acylrest steht,
$Y^I$ für die Gruppierung $OR^4$ oder $-NR^5R^6$ steht, in welcher
$R^4$ für Alkyl oder gegebenenfalls substituiertes Benzyl steht,
$R^5$ für Alkyl steht,
$R^6$ für Alkyl steht oder

$R^5$ und $R^6$ gemeinsam mit dem Stickstoffatom, an das sie gebunden sind, für einen gegebenenfalls substituierten mehrgliedrigen Ring stehen, der weitere gleiche oder verschiedene Heteroatome enthalten kann,

$X^I$ für Alkenyl, Alkinyl, Alkoxycarbonyl, Dialkylaminocarbonyl oder Cyano steht, erhält,

wenn man 1-Amino-cyclopropancarbonsäurederivate der Formel (II)

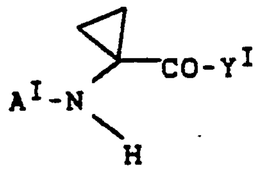

(II)

in welcher $A^I$ und $Y^I$ die oben angegebenen Bedeutungen haben, mit Verbindungen der Formel (III)

$Z-CH_2-(\underset{R}{C}H)_n-X^I$ (III)

in welcher R, n und $X^I$ die oben angegebenen Bedeutungen haben, und

Z für eine Abgangsgruppe steht,

in Gegenwart einer Base, gegebenenfalls in Gegenwart eines Lösungsmittels und gegebenenfalls in Gegenwart eines Phasentransferkatalysators umsetzt,

oder daß man

b) die erfindungsgemäßen substituierten 1-Amino-cyclopropancarbonsäurederivate der Formel (I-2)

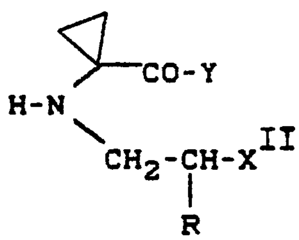

(I-2)

in welcher

R und Y die oben angegebene Bedeutungen haben, und

$X^{II}$ für Alkoxycarbonyl oder Cyano steht, erhält,

wenn man 1-Amino-cyclopropancarbonsäurederivate der Formel (IV)

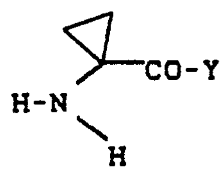

(IV)

in welcher Y die oben angegebene Bedeutung hat, mit Olefinen der Formel (V)

$H_2C=\underset{R}{C}H-X^{II}$ (V)

in welcher $X^{II}$ und R die oben angegebenen Bedeutungen haben,

gegebenenfalls in Gegenwart eines Lösungsmittels und gegebenenfalls in Gegenwart eines Katalysators umsetzt,

oder daß man

c) die erfindungsgemäßen substituierten 1-Amino-cyclopropancarbonsäurederivate der Formel (I-3)

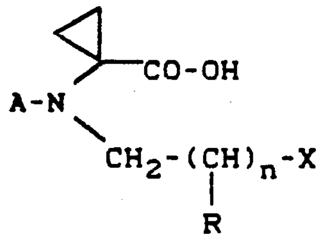

(I-3)

in welcher
A, R, n und X die oben angegebenen Bedeutungen haben, erhält, wenn man erfindungsgemäße 1-Amino-cyclopropancarbonsäurebenzylester der Formel (I-4)

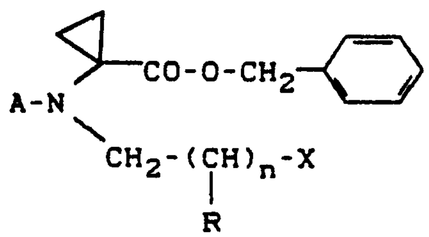

(I-4)

in welcher A, R, n und X die oben angegebenen Bedeutungen haben,
mit Wasserstoff in Gegenwart eines Katalysators und in Gegenwart eines Lösungsmittels hydriert,
oder daß man

d) die erfindungsgemäßen substituierten 1-Amino-cyclopropancarbonsäurederivate der Formel (I-5)

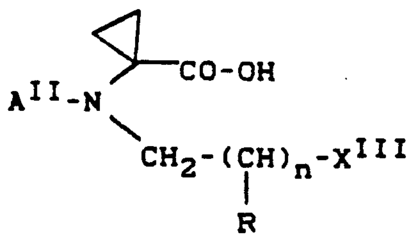

(I-5)

in welcher R und n die oben angegebenen Bedeutungen haben,
$A^{II}$ für die Bedeutung von A mit Ausnahme von Trifluoracetyl steht und
$X^{III}$ für die Bedeutung von X steht mit Ausnahme von primärem und sekundärem Alkoxycarbonyl, erhält, wenn man erfindungsgemäße substituierte 1-Amino-cyclopropancarbonsäureester der Formel (I-6)

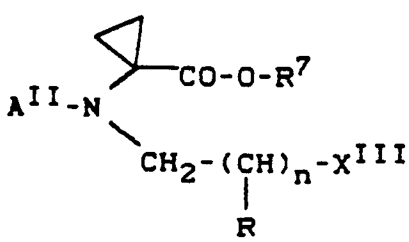

(I-6)

in welcher $A^{II}$, R, n und $X^{III}$ die oben angegebenen Bedeutungen haben und
$R^7$ für Methyl oder Ethyl steht,
in Gegenwart einer wäßrigen Base und gegebenenfalls eines Verdünnungsmittels hydrolysiert,
oder daß man

e) die erfindungsgemäßen substituierten 1-Amino-cyclopropancarbonsäurederivate der Formel (I-7)

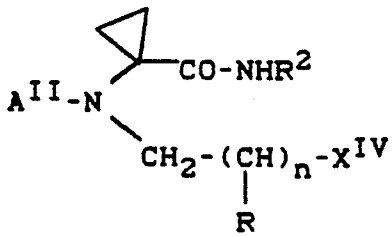

(I-7)

in welcher $A^{II}$, $R^2$, R und n die oben angegebenen Bedeutungen haben und $X^{IV}$ für die Bedeutung von X mit Ausnahme von primären Alkoxycarbonyl steht, erhält, wenn man erfindungsgemäße substituierte 1-Amino-cyclopropancarbonsäureester der Formel (I-8)

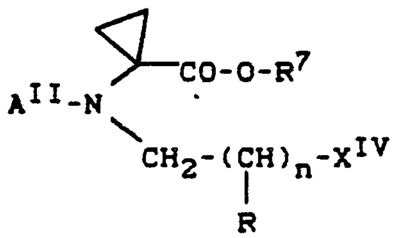

(I-8)

in welcher $A^{II}$, $R^7$, R, n und $X^{IV}$ die oben angegebenen Bedeutungen haben,
mit Aminen der Formel (VI)
$H_2NR^2$ (VI)
in welcher $R^2$ die oben angegebene Bedeutung hat,
in Gegenwart eines Lösungsmittels umsetzt,
oder daß man

f) die erfindungsgemäßen substituierten 1-Amino-cyclopropancarbonsäurederivate der Formel (I-9)

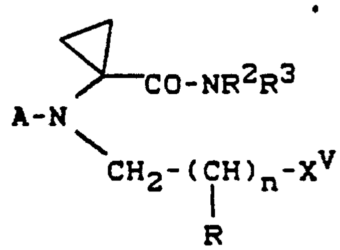

(I-9)

in welcher A, R, $R^2$, $R^3$ und n die oben angegebenen Bedeutungen haben und $X^V$ für die Bedeutung von X mit Ausnahme von Hydroxycarbonyl steht, erhält, wenn man carboxy-aktivierte Derivate der erfindungsgemäßen 1-Amino-cyclopropancarbonsäuren der Formel (I-10)

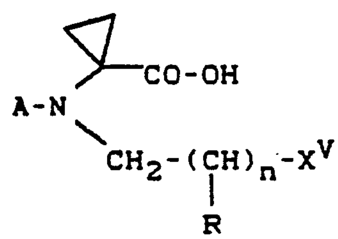

(I-10)

in welcher A, R, n und $X^V$ die oben angegebenen Bedeutungen haben,
mit Aminen der Formel (VII)
$HNR^2R^3$ (VII)
in welcher $R^2$ und $R^3$ die oben angegebenen Bedeutungen haben,

gegebenenfalls in Gegenwart einer Base, in Gegenwart eines Lösungsmittels und gegebenenfalls in Gegenwart eines Katalysators umsetzt,
oder daß man

g) die erfindungsgemäßen substituierten 1-Amino-cyclopropancarbonsäurederivate der Formel (I-11)

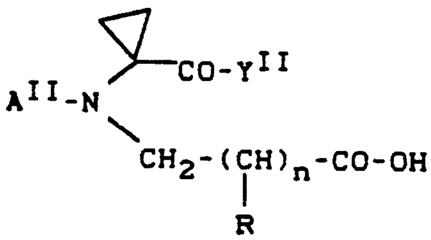

(I-11)

in welcher
$A^{II}$, R und n die oben angegebenen Bedeutungen haben und
$Y^{II}$ für tert.-Alkoxy oder die Gruppierung $NR^2R^3$ steht, wobei
$R^2$ und $R^3$ die oben angegebenen Bedeutungen haben, erhält,
wenn man die Ester der Formel (I-12)

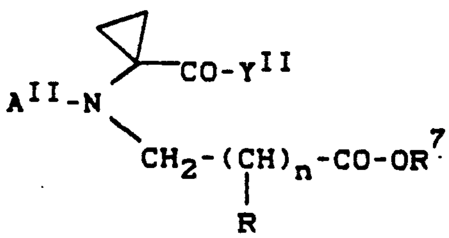

(I-12)

in welcher $A^{II}$, $Y^{II}$, R, n und $R^7$ die oben angegebenen Bedeutungen haben,
in Gegenwart einer wäßrigen Base und gegebenenfalls in Gegenwart eines Verdünnungsmittels hydrolysiert,
oder daß man

h) die erfindungsgemäßen substituierten 1-Amino-cyclopropancarbonsäurederivate der Formel (I-13)

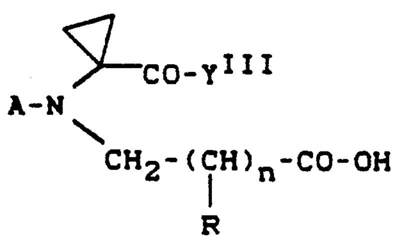

(I-13)

in welcher A, R und n die oben angegebenen Bedeutungen haben und
$Y^{III}$ für die Gruppierung $NR^2R^3$ oder $OR^5$ steht, in welchen $R^2$, $R^3$ und $R^5$ die oben angegebenen Bedeutungen haben, erhält,
wenn man die Benzylester der Formel (I-14)

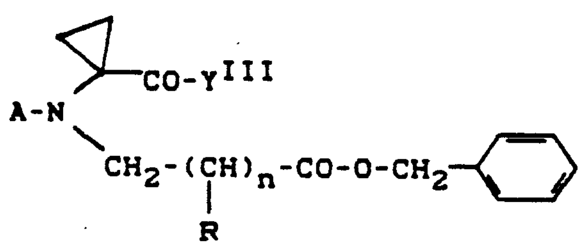

(I-14)

in welcher A, $Y^{III}$, R, und n die oben angegebenen Bedeutungen haben, mit Wasserstoff in Gegenwart eines Katalysators und und in Gegenwart eines Lösungsmittels hydriert, oder daß man

i) die erfindungsgemäßen substituierten 1-Amino-cyclopropancarbonsäurederivate der Formel (I-15)

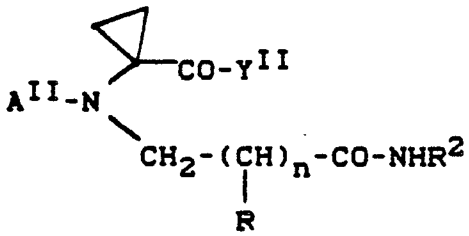

(I-15)

in welcher $A^{II}$, $Y^{II}$, R, n und $R^2$ die oben angegebenen Bedeutungen haben, erhält, wenn man die erfindungsgemäßen Ester der Formel (I-12)

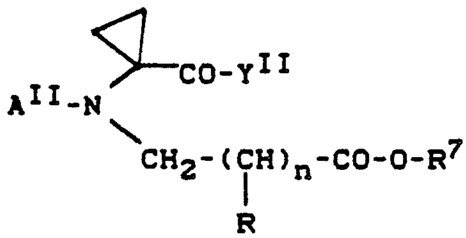

(I-12)

in welcher $A^{II}$, $Y^{II}$, R, n und $R^7$ die oben angegebenen Bedeutungen haben, mit Aminen der Formel (VI)

$H_2NR^2$ (VI)

in welcher $R^2$ die oben angegebene Bedeutung hat, in Gegenwart eines Lösungsmittels umsetzt, oder daß man

k) die erfindungsgemäßen substituierten 1-Amino-cyclopropancarbonsäurederivate der Formel (I-16)

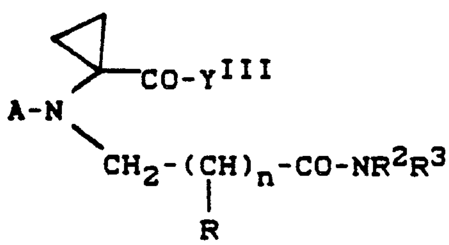

(I-16)

in welcher A, $Y^{III}$, R, n, $R^2$ und $R^3$ die oben angegebenen Bedeutungen haben, erhält, wenn man carboxy-aktivierte Derivate der erfindungsgemäßen Carbonsäuren der Formel (I-13)

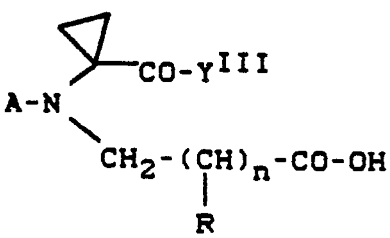

(I-13)

in welcher A, $Y^{III}$, R und n die oben angegebenen Bedeutungen haben,
mit Aminen der Formel (VII)
$HNR^2R^3$ (VII)
in welcher $R^2$ und $R^3$ die oben angegebenen Bedeutungen haben,
gegebenenfalls in Gegenwart einer Base, gegebenenfalls in Gegenwart eines Lösungsmittels und gegebenenfalls in Gegenwart eines Katalysators umsetzt,
oder daß man

l) die erfindungsgemäßen substituierten 1-Amino-cyclopropancarbonsäurederivate der Formel (I-17)

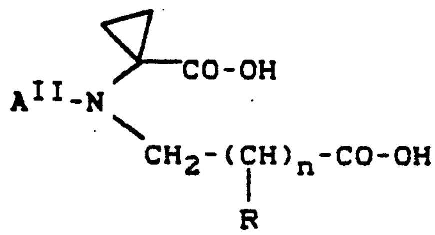
(I-17)

in welcher $A^{II}$, R und n die oben angegebenen Bedeutungen haben, erhält,
wenn man die erfindungsgemäßen Diester der Formel (I-18)

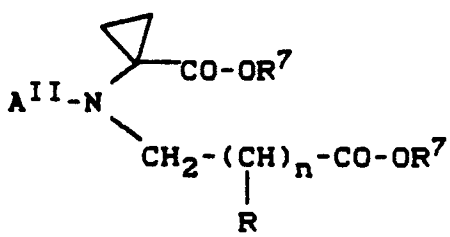
(I-18)

in Gegenwart einer wäßrigen Base und gegebenenfalls in Gegenwart eines Verdünnungsmittels hydrolisiert,
oder daß man

m) die erfindungsgemäßen substituierten 1-Amino-cyclopropancarbonsäurederivate der Formel (I-19)

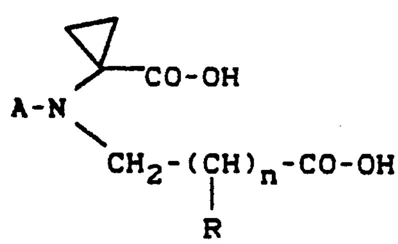
(I-19)

in welcher A, R und n die oben angegebenen Bedeutungen haben, erhält,
wenn man die Dibenzylester der Formel (I-20)

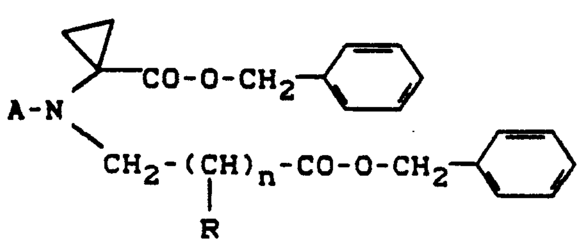
(I-20)

in welcher A, R und n die oben angegebenen Bedeutungen haben,
mit Wasserstoff in Gegenwart eines Katalysators und in Gegenwart eines Lösungsmittels hydriert,
oder daß man

n) die erfindungsgemäßen substituierten 1-Amino-cyclopropancarbonsäurederivate der Formel (I-21)

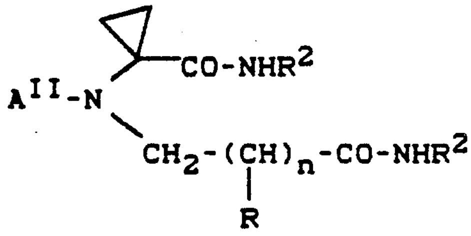

(I-21)

in welcher $A^{II}$, $R^2$, R und n die oben angegebenen Bedeutungen haben, erhält,
wenn man die erfindungsgemäßen Diester der Formel (I-18)

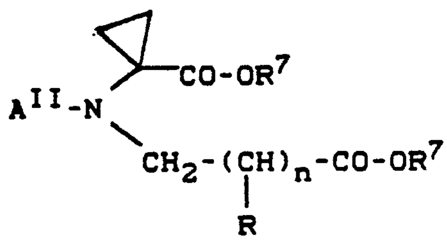

(I-18)

in welcher $A^{II}$, $R^7$, R und n die oben angegebenen Bedeutungen haben,
mit Aminen der Formel (VI)
$H_2NR^2$ (VI)
in welcher $R^2$ die oben angegebene Bedeutung hat,
in Gegenwart eines Lösungsmittels umsetzt,
oder daß man

o) die erfindungsgemäßen substituierten 1-Amino-cyclopropancarbonsäurederivate der Formel (I-22)

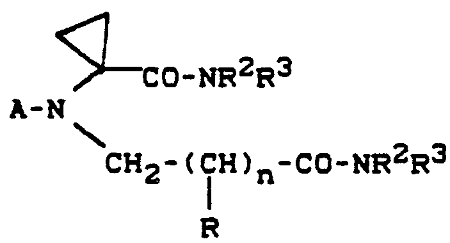

(I-22)

in welcher A, $R^2$, $R^3$, R und n die oben angegebenen Bedeutungen haben, erhält,
wenn man carboxy-aktivierte Derivate der erfindungsgemäßen Dicarbonsäuren der Formel (I-19)

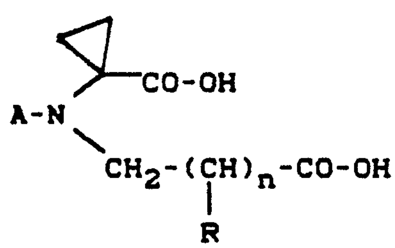

(I-19)

in welcher A, R und n die oben angegebenen Bedeutungen haben,
mit Aminen der Formel (VII)
$HNR^2R^3$ (VII)
in welcher $R^2$ und $R^3$ die oben angegebenen Bedeutungen haben,

gegebenenfalls in Gegenwart einer Base, in Gegenwart eines Lösungsmittels und gegebenenfalls in Gegenwart eines Katalysators umsetzt,
oder daß man

p) die erfindungsgemäßen substituierten 1-Amino-cyclopropancarbonsäurederivate der Formel (I-23)

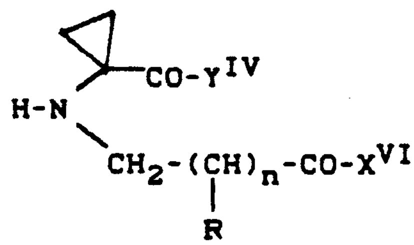

(I-23)

in welcher
R und n die oben angegebenen Bedeutungen haben,
$Y^{IV}$ für die Bedeutung von Y mit Ausnahme von tert.-Alkoxy steht, und
$X^{VI}$ für die Bedeutung von X mit Ausnahme von tert.-Alkoxycarbonyl steht, erhält,
wenn man die erfindungsgemäßen Formylderivate der Formel (I-24)

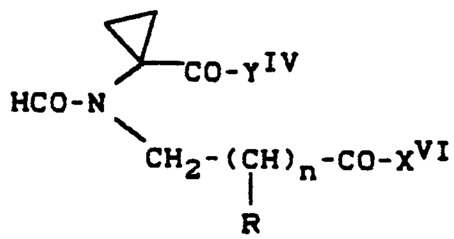

(I-24)

in welcher $Y^{IV}$, R, n und $X^{VI}$ die oben angegebenen Bedeutungen haben,
mit einer Säure in Gegenwart eines Lösungsmittels behandelt,
oder daß man

q) die erfindungsgemäßen substituierten 1-Amino-cyclopropancarbonsäurederivate der Formel (I-25)

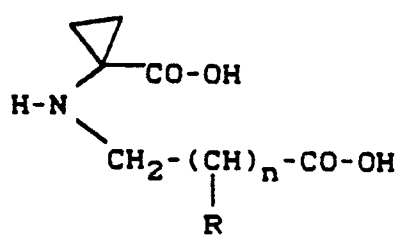

(I-25)

in welcher
R und n die oben angegebenen Bedeutungen haben, erhält,
wenn man die erfindungsgemäßen Formylderivate der Formel (I-26)

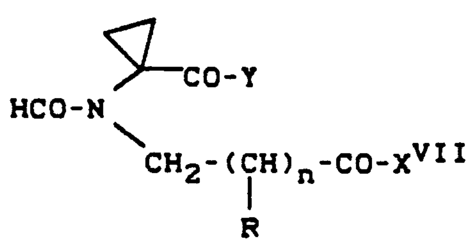

(I-26)

in welcher Y, R und n die oben angegebenen Bedeutungen haben und
$X^{VII}$ für Hydroxycarbonyl, Alkoxycarbonyl, Aminocarbonyl, Alkylaminocarbonyl, Dialkylaminocarbonyl oder Cyano steht,
mit wäßrigen Mineralsäuren gegebenenfalls in Gegenwart eines Lösungsmittels erhitzt,
oder daß man

r) die erfindungsgemäßen substituierten 1-Amino-cyclopropancarbonsäurederivate der Formel (I-27)

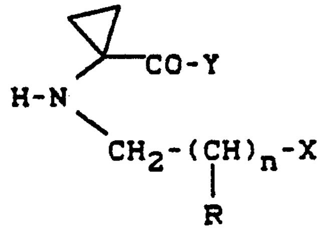

(I-27)

in welcher Y, R, n und X die oben angegebenen Bedeutungen haben, erhält,
wenn man die Trifluoracetylderivate der Formel (I-28)

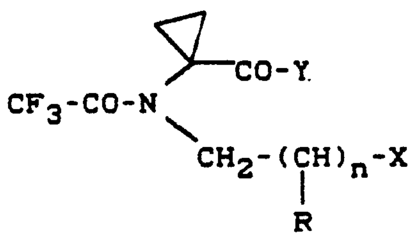

(I-28)

mit einer Base in Gegenwart eines Verdünnungsmittels umsetzt,
oder daß man

s) die erfindungsgemäßen substituierten 1-Amino-cyclopropancarbonsäurederivate der Formel (I-29)

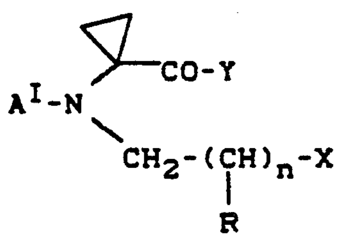

(I-29)

in welcher $A^I$, Y, R, n und X die oben angegebenen Bedeutungen haben, erhält,
wenn man die erfindungsgemäßen Verbindungen der Formel (I-27)

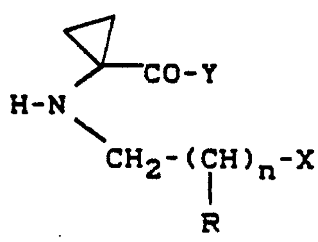

(I-27)

in welcher Y, R, n und X die oben angegebenen Bedeutungen haben,
mit carboxy-aktivierten Derivaten der Carbonsäuren der Formel (VIII)
$A^I$-OH (VIII)
in welcher $A^I$ die oben angegebene Bedeutung hat,
gegebenenfalls in Gegenwart einer Base, in Gegenwart eines Lösungsmitels und gegebenenfalls in

Gegenwart eines Katalysators acyliert und gegebenenfalls die so erhaltenen Verbindungen der Formel (I) weiter umsetzt mit Aminen und Alkali-bzw, Erdalkalihydroxiden, -carbonaten oder -hydrogencarbonaten, Ammoniumhydroxid oder pflanzenverträglichen Säuren addiert.

6. Schädlingsbekämpfungsmittel, gekennzeichnet durch einen Gehalt an mindestens einem substituierten 1-Amino-cyclopropancarbonsäurederivat der Formel (I) gemäß den Ansprüchen 1-5.

7. Verwendung von substituierten 1-Amino-cyclopropancarbonsäurederivaten der Formel (I) gemäß den Ansprüchen 1-5 zur Bekämpfung von Schädlingen.

8. Verfahren zur Bekämpfung von Schädlingen, dadurch gekennzeichnet, daß man substituierte 1-Amino-cyclopropancarbonsäurederivate der Formel (I) gemäß den Ansprüchen 1-5 auf Schädlinge oder ihren Lebensraum einwirken läßt.

9. Verfahren zur Herstellung von Schädlingsbekämpfungsmitteln, dadurch gekennzeichnet, daß man substituierte 1-Amino-cyclopropancarbonsäurederivate der Formel (I) gemäß den Ansprüchen 1-5 mit Streckmitteln und/oder oberflächenaktiven Mitteln vermischt.

10. Verwendung von substituierten 1-Amino-cyclopropancarbonsäurederivaten der Formel (I) gemäß den Ansprüchen 1-5 zur Bekämpfung von Pilzen.

Europäisches Patentamt

# EUROPÄISCHER RECHERCHENBERICHT

Nummer der Anmeldung

EP 86114988.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 136 615 (BAYER)<br>* Ansprüche * | 1,5-7 | C 07 C 121/43<br>C 07 C 103/46 |
| D,A | & DE-A-3 335 514<br>-- | | C 07 C 103/737<br>C 07 C 101/36 |
| A | EP - A1 - 0 051 884 (SHELL)<br>* Ansprüche *<br>-- | 1,5-7 | C 07 C 120/00<br>C 07 C 102/00<br>C 07 C 99/00 |
| A | EP - A2 - 0 066 760 (BAYER)<br>* Ansprüche *<br>---- | 1,5-7 | C 07 D 307/30<br>A 01 N 37/00 |
| | | | **RECHERCHIERTE SACHGEBIETE (Int. Cl.4)** |
| | | | C 07 C 101/00<br>C 07 C 103/00<br>C 07 C 121/00 |

Der vorliegende Recherchenbericht wurde für alle Patentansprüche erstellt.

| Recherchenort | Abschlußdatum der Recherche | Prüfer |
|---|---|---|
| WIEN | 21-01-1987 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82